# EUROPEAN PATENT SPECIFICATION

(11) **EP 2 045 328 B1**
(45) Date of publication and mention of the grant of the patent: **08.02.2012**
(21) Application number: 07803657.1
(22) Date of filing: 12.07.2007
(51) Int. Cl.: C12N 15/13, C12N 5/18, C12P 21/08, A61K 39/00, G01N 33/577

(54) **PEPTIDE AND NUCLEOTIDE SEQUENCES OF ANISAKIS SPP. AND USES THEREOF**
PEPTID- UND NUKLEOTIDSEQUENZEN VON ANISAKIS SPP. UND IHRE VERWENDUNG
SEQUENCES PEPTIDIQUES ET NUCLEOTIDIQUES D'ANISAKIS SPP. ET LEURS APPLICATIONS

(30) Priority: 13.07.2006 ES 200601879
(43) Date of publication of application: 08.04.2009
(73) Proprietor: MARTINEZ UBEIRA, Florencio, 15782 Santiago de Compostela (ES)
(72) Inventor: MARTÍNEZ UBEIRA, Florencio, E-15782 Santiago De Compostela (ES); GÁRATE ORMAECHEA, María Teresa, E-15782 Santiago De Compostela (ES)
(74) Representative: Pons Ariño, Angel
(86) International application number: PCT/ES2007/070130
(87) International publication number: WO 2008/006927

(56) References cited:
- DATABASE Geneseq [Online] 1 December 2005 (2005-12-01), "Human cDNA clone protein DFNES20055270, SEQ ID 3175." XP002577052 retrieved from EBI accession no. GSP:AEC87420 Database accession no. AEC87420
- IGLESIAS R: "Monoclonal Antibodies against diagnostic Anisakis simplex antigens" PARASITOLOGY RESEARCH, SPRINGER VERLAG, BERLIN, DE LNKD- DOI:10.1007/S004360050335, vol. 83, no. 8, 1 September 1997 (1997-09-01), pages 755-761, XP008102060 ISSN: 0932-0113
- LORENZO S ET AL: "Usefulness of currently available methods for the diagnosis of Anisakis simplex allergy" ALLERGY (COPENHAGEN), vol. 55, no. 7, July 2000 (2000-07), pages 627-633, XP007912523 ISSN: 0105-4538
- IGLESIAS R.: 'Monoclonal Antibodies against diagnostic Anisakis simplex antigens' PARASITOLOGY RESEARCH vol. 83, 1997, pages 755 - 761, XP008102060
- PEREZ-PEREZ J. ET AL.: 'Molecular Cloning of Paramyosin, a New Allergen of Anisakis simplex' ALLERGY AND IMMUNOLOGY vol. 123, 2000, pages 120 - 129, XP008102049
- LORENZO S. ET AL.: 'O-glicans as a source of cross-reactivity in determinations of human serum antibodies to Anisakis simplex antigens' CLINICAL AND EXPERIMENTAL ALLERGY vol. 30, 2000, pages 551 - 559, XP008102061

### SEQUENCE LISTING

<110> Universidade de Santiago de Compostela
<120> PEPTIDE AND NUCLEOTIDE SEQUENCES OF ANISAKIS spp., ANTIBODIES THAT RECOGNISE SAID SEQUENCESAND USES OF SAME
<130> EP1596.2
<160> 28
<170> PatentIn version 3.3
<210> 1
   <211> 3288
   <212> DNA
   <213> Anisakis spp.
<220>
   <221> misc_feature
   <222> (1)..(3288)
   <223> Encoding region of the antigenic sequenceb SEQ ID n° 2 of Anisakis spp.
<400> 1
   gcgaaatacg gaagccaatt ctgtaagaat cttcttgcaa attgcttgtc ttctacgggt 60
   gcaacactac cgatgcaatc accatggcag ataccgccgg ttgtttcttc atgcataacc 120
   tcaggaatgg cgaaaggaac tgaccataat aaaaaagacg tgatggcaac gtgcatacaa 180
   agatatggtg cagacttttg taagaacatg gtgggatcat gcgctgcatt gactgatgtg 240
   actcttacat cttatggaat tggttcagta ctaccacaag ttattgtcga ctgcatgacc 300
   tcggagatgt ctagtccgag cataatgtgg caatgtgtgc agaagtatgg tactgaattt 360
   tgcaagaaac tgctgcaaga ttgttctgct tctacgggtg catccttatc tccccaagca 420
   ccttggctca taccaagtgt gatcgctgaa tgtatggcca aaggtatggt caatggagga 480
   cggcaagctg atgacacaat ggcaatttgc atccaaaaat acggcatcaa attttgcaac 540
   atcataggag ctgcgtgctc agtgttaaca caagtgccat tcttcccaca actgcctggg 600
   actgtgcagc aattaccatc agaacttcgt gcatgcgtgc gatcggagac ccaaaaacca 660
   aacgtgatga ccaaatgcgt ggagaaatat ggcacggaat tttgtagtag cctgttgcaa 720
   agttgttctg catcaaccgg tgcaatatta cctctgcgag aaccgtggaa gataccgcac 780
   ccgattgcag actgtatgta tgcaggtatg aatccagaat ctaaagaaga ccgtggaggt 840
   ataatgtcga aatgcatcag acgttatggt tttgatttct gcaagaagat gttggaatcc 900
   tgttcggcat taactagtgt acagcatgac tcacgaaaca ctaactacgc atcacttcca 960
   caagtattga aggattgtat ggcatcagaa atggatagtc cgagcgtaat gttcgaatgc 1020
   gtgcaaagat atggtacacc attttgcaag ggcctcttgg aaacatgcac tgagaaaaca 1080
   cgggcatccc tctcaccaca ggcgccgtgg ttgataccaa ctgcaattgc acaatgcatg 1140
   cgtcaaggaa tgaaccaagg agatgaaaag aagacattga tgtcgttgtg tgtaagaaga 1200
   tatggggctg attactgcaa caatctggca gcagcctgtt ctgttcttac caatttaccg 1260
   ttcttcccac agacacctcc atctgaacaa aatctaccac caatgatgca caaatgctta 1320
   aagtcggaaa tggaaaatcc tggtattatg tccacatgtg ttcaaagata tggtactcag 1380
   ttttgcaaga acttactgaa ctcgtgcaca gcgtcaaccg gtatgcatct tccgtacgat 1440
   gcaccttgga aaatacccga gccaatcgct gcttgcatgg cccaaggaat gaatggtggt 1500
   gaccacaaac ctaaggagga tgcgatggcg agatgcatac gtaaatatgg tgtcacttac 1560
   tgcaacaaca tgctagcatc ttgttctgta ttgaccaatg taaattatga ccctagtggc 1620
   ggtcaaatgc cgggaatatt atcggaatgc gtgactgcag aaacggatga accgagtgca 1680
   atgtgccaat gcgttcaaaa gtacggtact gaattctgca agaaacgcct tgcatcgtgt 1740
   attgcttcta cgggtatgaa tcttcccgcc actacaccat ggaaactgcc tccacctgtg 1800
   gctcgctgca tgcagcacgg gtcacctaat gacaaccgcg gacagggaca gggttcaaat 1860
   gacgtaatgt ctcaatgtat tgctagatac ggagccgaat tttgtcaacg gttagcgaga 1920
   ttctgttatg caatgaacag tttacaatat ccaggagaga cttttgatcc acagcaacag 1980
   actccaacac aagtagcaag atgcatgaag tcagaaatgg atagtccgtc cgtaatgtgg 2040
   cactgtgtgc agaaatatgg tcaagaattt tgtaacaagt tagctgccac ctgttcgaca 2100
   gaaacgaaca cacctcttcc gcaacaggat ccttggcgat taccgcaacc aataatcgcc 2160
   tgcatgttag gaaaaatgaa taatcctaat ccaacatcca aacctcagag tgtaatgtca 2220
   cagtgcacag ccagatatgg tgatgatttc tgcttgagtc tcggaaaagc ttgtgctgaa 2280
   ctcaacaatg taccatcctc catgataagt ctttctgcac agcaattacc ccaaccagtt 2340
   tccagttgta tgaaagcgga aatgaacaac ccgagtgcac tgtggcagtg tatacagcaa 2400
   tacggcatag aattttgcaa gaaattaaga gatgcttgtt ctgcaatgac tggcgcatct 2460
   ctttccacta caactccttg gattttgccg caaccagtat caaactgtat gagaaacgag 2520
   atgaataatc caagtgcaat gtggctttgc atccagaaat atggcattga attctgcaat 2580
   agactagcgt cggcttgtgc gatgatcaag aaggttacta tgcccacagt gaccataaat 2640
   ctaccagaaa taatagcaag ctgcgtcgct tcagagaatt cgcaggcaat gtgctacgcc 2700
   cgtaaaggac ctgaacaatg taaaacagaa gaaaacatct gtcggaatcc gaacaatcct 2760
   ccagggagcc cgttgactat tccggagacg gagtgcatga agagtcaggt tgcaatggcc 2820
   acatgccaga agaaattcgg atcagagtgt gttgcgcttc aacaagaatg tgttgctgga 2880
   actggagcgc ctcctgttac gattggtgcg agaggagcgt tcatgttggc cacagcactt 2940
   cgttcatgta tcttcaacgg aggcgttatt ggtagttgtg ttctgtatca cccaccatct 3000
   caatgtgacc agtgggtcca gcaatgtgcc actgcactgc aaacatcagc tggtgtaact 3060
   gttgcgggcg gttatcgcca gctatctccc ccgatggcag tatgtgtcgc atcacaggat 3120
   ttgatgacaa gatgcatgac gagattgggc caaggaactt gtcaacaggc agtcaaaaac 3180
   tgcaagcgaa gattcaacac accttcttca agacttccgg gaaggttgtg gtcgctgtcg 3240
   tcagaactaa tcaattgctt atatcgtcct gtgaacagag ctagcaac               3288
<210> 2
   <211> 1096
   <212> PRT
   <213> Anisakis spp.
<220>
   <221> Peptide
   <222> (1)..(1096)
   <223> Aminoacidic sequence of SEQ ID No 1
<400> 2
   Ala Lys Tyr Gly Ser Gln Phe Cys Lys Asn Leu Leu Ala Asn Cys Leu
   1               5                   10                  15
   Ser Ser Thr Gly Ala Thr Leu Pro Met Gln Ser Pro Trp Gln Ile Pro
               20                  25                  30
   Pro Val Val Ser Ser Cys Ile Thr Ser Gly Met Ala Lys Gly Thr Asp
           35                  40                  45
   His Asn Lys Lys Asp Val Met Ala Thr Cys Ile Gln Arg Tyr Gly Ala
       50                  55                  60
   Asp Phe Cys Lys Asn Met Val Gly Ser Cys Ala Ala Leu Thr Asp Val
   65                  70                  75                  80
   Thr Leu Thr Ser Tyr Gly Ile Gly Ser Val Leu Pro Gln Val Ile Val
                   85                  90                  95
   Asp Cys Met Thr Ser Glu Met Ser Ser Pro Ser Ile Met Trp Gln Cys
               100                 105                 110
   Val Gln Lys Tyr Gly Thr Glu Phe Cys Lys Lys Leu Leu Gln Asp Cys
           115                 120                 125
   Ser Ala Ser Thr Gly Ala Ser Leu Ser Pro Gln Ala Pro Trp Leu Ile
       130                 135                 140
   Pro Ser Val Ile Ala Glu Cys Met Ala Lys Gly Met Val Asn Gly Gly
   145                 150                 155                 160
   Arg Gln Ala Asp Asp Thr Met Ala Ile Cys Ile Gln Lys Tyr Gly Ile
                   165                 170                 175
   Lys Phe Cys Asn Ile Ile Gly Ala Ala Cys Ser Val Leu Thr Gln Val
               180                 185                 190
   Pro Phe Phe Pro Gln Leu Pro Gly Thr Val Gln Gln Leu Pro Ser Glu
           195                 200                 205
   Leu Arg Ala Cys Val Arg Ser Glu Thr Gln Lys Pro Asn Val Met Thr
       210                 215                 220
   Lys Cys Val Glu Lys Tyr Gly Thr Glu Phe Cys Ser Ser Leu Leu Gln
   225                 230                 235                 240
   Ser Cys Ser Ala Ser Thr Gly Ala Ile Leu Pro Leu Arg Glu Pro Trp
                   245                 250                 255
   Lys Ile Pro His Pro Ile Ala Asp Cys Met Tyr Ala Gly Met Asn Pro
               260                 265                 270
   Glu Ser Lys Glu Asp Arg Gly Gly Ile Met Ser Lys Cys Ile Arg Arg
           275                 280                 285
   Tyr Gly Phe Asp Phe Cys Lys Lys Met Leu Glu Ser Cys Ser Ala Leu
       290                 295                 300
   Thr Ser Val Gln His Asp Ser Arg Asn Thr Asn Tyr Ala Ser Leu Pro
   305                 310                 315                 320
   Gln Val Leu Lys Asp Cys Met Ala Ser Glu Met Asp Ser Pro Ser Val
                   325                 330                 335
   Met Phe Glu Cys Val Gln Arg Tyr Gly Thr Pro Phe Cys Lys Gly Leu
               340                 345                 350
   Leu Glu Thr Cys Thr Glu Lys Thr Arg Ala Ser Leu Ser Pro Gln Ala
           355                 360                 365
   Pro Trp Leu Ile Pro Thr Ala Ile Ala Gln Cys Met Arg Gln Gly Met
       370                 375                 380
   Asn Gln Gly Asp Glu Lys Lys Thr Leu Met Ser Leu Cys Val Arg Arg
   385                 390                 395                 400
   Tyr Gly Ala Asp Tyr Cys Asn Asn Leu Ala Ala Ala Cys Ser Val Leu
                   405                 410                 415
   Thr Asn Leu Pro Phe Phe Pro Gln Thr Pro Pro Ser Glu Gln Asn Leu
               420                 425                 430
   Pro Pro Met Met His Lys Cys Leu Lys Ser Glu Met Glu Asn Pro Gly
           435                 440                 445
   Ile Met Ser Thr Cys Val Gln Arg Tyr Gly Thr Gln Phe Cys Lys Asn
       450                 455                 460
   Leu Leu Asn Ser Cys Thr Ala Ser Thr Gly Met His Leu Pro Tyr Asp
   465                 470                 475                 480
   Ala Pro Trp Lys Ile Pro Glu Pro Ile Ala Ala Cys Met Ala Gln Gly
                   485                 490                 495
   Met Asn Gly Gly Asp His Lys Pro Lys Glu Asp Ala Met Ala Arg Cys
               500                 505                 510
   Ile Arg Lys Tyr Gly Val Thr Tyr Cys Asn Asn Met Leu Ala Ser Cys
           515                 520                 525
   Ser Val Leu Thr Asn Val Asn Tyr Asp Pro Ser Gly Gly Gln Met Pro
       530                 535                 540
   Gly Ile Leu Ser Glu Cys Val Thr Ala Glu Thr Asp Glu Pro Ser Ala
   545                 550                 555                 560
   Met Cys Gln Cys Val Gln Lys Tyr Gly Thr Glu Phe Cys Lys Lys Arg
                   565                 570                 575
   Leu Ala Ser Cys Ile Ala Ser Thr Gly Met Asn Leu Pro Ala Thr Thr
               580                 585                 590
   Pro Trp Lys Leu Pro Pro Pro Val Ala Arg Cys Met Gln His Gly Ser
           595                 600                 605
   Pro Asn Asp Asn Arg Gly Gln Gly Gln Gly Ser Asn Asp Val Met Ser
       610                 615                 620
   Gln Cys Ile Ala Arg Tyr Gly Ala Glu Phe Cys Gln Arg Leu Ala Arg
   625                 630                 635                 640
   Phe Cys Tyr Ala Met Asn Ser Leu Gln Tyr Pro Gly Glu Thr Phe Asp
                   645                 650                 655
   Pro Gln Gln Gln Thr Pro Thr Gln Val Ala Arg Cys Met Lys Ser Glu
               660                 665                 670
   Met Asp Ser Pro Ser Val Met Trp His Cys Val Gln Lys Tyr Gly Gln
           675                 680                 685
   Glu Phe Cys Asn Lys Leu Ala Ala Thr Cys Ser Thr Glu Thr Asn Thr
       690                 695                 700
   Pro Leu Pro Gln Gln Asp Pro Trp Arg Leu Pro Gln Pro Ile Ile Ala
   705                 710                 715                 720
   Cys Met Leu Gly Lys Met Asn Asn Pro Asn Pro Thr Ser Lys Pro Gln
                   725                 730                 735
   Ser Val Met Ser Gln Cys Thr Ala Arg Tyr Gly Asp Asp Phe Cys Leu
               740                 745                 750
   Ser Leu Gly Lys Ala Cys Ala Glu Leu Asn Asn Val Pro Ser Ser Met
           755                 760                 765
   Ile Ser Leu Ser Ala Gln Gln Leu Pro Gln Pro Val Ser Ser Cys Met
       770                 775                 780
   Lys Ala Glu Met Asn Asn Pro Ser Ala Leu Trp Gln Cys Ile Gln Gln
   785                 790                 795                 800
   Tyr Gly Ile Glu Phe Cys Lys Lys Leu Arg Asp Ala Cys Ser Ala Met
                   805                 810                 815
   Thr Gly Ala Ser Leu Ser Thr Thr Thr Pro Trp Ile Leu Pro Gln Pro
               820                 825                 830
   Val Ser Asn Cys Met Arg Asn Glu Met Asn Asn Pro Ser Ala Met Trp
           835                 840                 845
   Leu Cys Ile Gln Lys Tyr Gly Ile Glu Phe Cys Asn Arg Leu Ala Ser
       850                 855                 860
   Ala Cys Ala Met Ile Lys Lys Val Thr Met Pro Thr Val Thr Ile Asn
   865                 870                 875                 880
   Leu Pro Glu Ile Ile Ala Ser Cys Val Ala Ser Glu Asn Ser Gln Ala
                   885                 890                 895
   Met Cys Tyr Ala Arg Lys Gly Pro Glu Gln Cys Lys Thr Glu Glu Asn
               900                 905                 910
   Ile Cys Arg Asn Pro Asn Asn Pro Pro Gly Ser Pro Leu Thr Ile Pro
           915                 920                 925
   Glu Thr Glu Cys Met Lys Ser Gln Val Ala Met Ala Thr Cys Gln Lys
       930                 935                 940
   Lys Phe Gly Ser Glu Cys Val Ala Leu Gln Gln Glu Cys Val Ala Gly
   945                 950                 955                 960
   Thr Gly Ala Pro Pro Val Thr Ile Gly Ala Arg Gly Ala Phe Met Leu
                   965                 970                 975
   Ala Thr Ala Leu Arg Ser Cys Ile Phe Asn Gly Gly Val Ile Gly Ser
               980                 985                 990
   Cys Val Leu Tyr His Pro Pro Ser  Gln Cys Asp Gln Trp  Val Gln Gln
           995                 1000                 1005
   Cys Ala  Thr Ala Leu Gln Thr  Ser Ala Gly Val Thr  Val Ala Gly
       1010                 1015                 1020
   Gly Tyr  Arg Gln Leu Ser Pro  Pro Met Ala Val Cys  Val Ala Ser
       1025                 1030                 1035
   Gln Asp  Leu Met Thr Arg Cys  Met Thr Arg Leu Gly  Gln Gly Thr
       1040                 1045                 1050
   Cys Gln Gln Ala Val Lys Asn Cys Lys Arg Arg Phe Asn Thr Pro
       1055                1060                1065
   Ser Ser Arg Leu Pro Gly Arg Leu Trp Ser Leu Ser Ser Glu Leu
       1070                1075                1080
   Ile Asn Cys Leu Tyr Arg Pro Val Asn Arg Ala Ser Asn
       1085                1090                1095
<210> 3
   <211> 915
   <212> DNA
   <213> Anisakis spp.
<220>
   <221> misc_feature <222> (1)..(915)
   <223> Fragment of SEQ ID 1
<400> 3
   atgagaggat ctcaccatca ccatcaccat acggatccgc atgcgagctc ggtaccccgg     60
   gtcgacatga tgcacaaatg cttaaagtcg gaaatggaaa atcctggtat tatgtccaca    120
   tgtgttcaaa gatatggtac tcagttttgc aagaacttac tgaactcgtg cacagcgtca    180
   accggtatgc atcttccgta cgatgcacct tggaaaatac ccgagccaat cgctgcttgc    240
   atggcccaag gaatgaatgg tggtgaccac aaacctaagg aggatgcgat ggcgagatgc    300
   atacgtaaat atggtgtcac ttactgcaac aacatgctag catcttgttc tgtattgacc    360
   aatgtaaatt atgaccctag tggcggtcaa atgccgggaa tattatcgga atgcgtgact    420
   gcagaaacgg atgaaccgag tgcaatgtgc caatgcgttc aaaagtacgg tactgaattc    480
   tgcaagaaac gccttgcatc gtgtattgct tctacgggta tgaatcttcc cgccactaca    540
   ccatggaaac tgcctccacc tgtggctcgc tgcatgcagc acgggtcacc taatgacaac    600
   cgcggacagg gacagggttc aaatgacgta atgtctcaat gtattgctag atacggagcc    660
   gaattttgtc aacggttagc gagattctgt tatgcaatga acagtttaca atatccagga    720
   gagacttttg atccacagca acagactcca acacaagtag caagatgcat gaagtcagaa    780
   atggatagtc cgtccgtaat gtggcactgt gtgcagaaat atggtcaaga attttgtaac    840
   aagttagctg ccacctgttc gacagaaacg aacacacctc ttccgcaaca ggatccttgg    900
   cgaagcttaa ttagc                                                     915
<210> 4
   <211> 305
   <212> PRT
   <213> Anisakis spp.
<220>
   <221> PEPTIDE
   <222> (1)..(305)
   <223> Aminoacidic Sequence of SEQ ID 3
<400> 4
   Met Arg Gly Ser His His His His His His Thr Asp Pro His Ala Ser
   1               5                   10                  15
   Ser Val Pro Arg Val Asp Met Met His Lys Cys Leu Lys Ser Glu Met
               20                  25                  30
   Glu Asn Pro Gly Ile Met Ser Thr Cys Val Gln Arg Tyr Gly Thr Gln
           35                  40                  45
   Phe Cys Lys Asn Leu Leu Asn Ser Cys Thr Ala Ser Thr Gly Met His
       50                  55                  60
   Leu Pro Tyr Asp Ala Pro Trp Lys Ile Pro Gln Pro Ile Ala Ala Cys
   65                  70                  75                  80
   Met Ala Gln Gly Met Asn Gly Gly Asp His Lys Pro Lys Glu Asp Ala
                   85                  90                  95
   Met Ala Arg Cys Ile Arg Lys Tyr Gly Val Thr Tyr Cys Asn Asn Met
               100                 105                 110
   Leu Ala Ser Cys Ser Val Leu Thr Asn Val Asn Tyr Asp Pro Ser Gly
           115                 120                 125
   Gly Gln Met Pro Gly Ile Leu Ser Glu Cys Val Thr Ala Glu Thr Asp
       130                 135                 140
   Glu Pro Ser Ala Met Cys Gln Cys Val Gln Lys Tyr Gly Thr Glu Phe
   145                 150                 155                 160
   Cys Asn Lys Arg Leu Ala Ser Cys Ile Ala Ser Thr Gly Met Asn Leu
                   165                 170                 175
   Pro Ala Thr Thr Pro Trp Lys Leu Pro Pro Pro Val Ala Arg Cys Met
               180                 185                 190
   Gln His Gly Ser Pro Asn Asp Asn Arg Gly Gln Gly Gln Gly Ser Asn
           195                 200                 205
   Asp Val Met Ser Gln Cys Ile Ala Arg Tyr Gly Ala Glu Phe Cys Gln
       210                 215                 220
   Arg Leu Ala Arg Phe Cys Tyr Ala Met Asn Ser Leu Gln Tyr Pro Gly
   225                 230                 235                 240
   Glu Thr Phe Asp Pro Gln Gln Gln Thr Pro Thr Gln Val Ala Arg Cys
                   245                 250                 255
   Met Lys Ser Glu Met Asp Ser Pro Ser Val Met Trp His Cys Val Gln
               260                 265                 270
   Lys Tyr Gly Gln Glu Phe Cys Asn Lys Leu Ala Ala Thr Cys Ser Thr
           275                 280                 285
   Glu Thr Asn Thr Pro Leu Pro Gln Gln Asp Pro Trp Arg Ser Leu Ile
       290                 295                 300
   Ser
   305
<210> 5
   <211> 846
   <212> DNA
   <213> Anisakis spp
<220>
   <221> misc_feature
   <222> (1)..(846)
   <223> Fragment of SEQ ID 1
<400> 5
   atgcacaaat gcttaaagtc ggaaatggaa aatcctggta ttatgtccac atgtgttcaa      60
   agatatggta ctcagttttg caagaactta ctgaactcgt gcacagcgtc aaccggtatg     120
   catcttccgt acgatgcacc ttggaaaata cccgagccaa tcgctgcttg catggcccaa     180
   ggaatgaatg gtggtgacca caaacctaag gaggatgcga tggcgagatg catacgtaaa     240
   tatggtgtca cttactgcaa caacatgcta gcatcttgtt ctgtattgac caatgtaaat     300
   tatgacccta gtggcggtca aatgccggga atattatcgg aatgcgtgac tgcagaaacg     360
   gatgaaccga gtgcaatgtg ccaatgcgtt caaaagtacg gtactgaatt ctgcaagaaa     420
   cgccttgcat cgtgtattgc ttctacgggt atgaatcttc ccgccactac accatggaaa     480
   ctgcctccac ctgtggctcg ctgcatgcag cacgggtcac ctaatgacaa ccgcggacag     540
   ggacagggtt caaatgacgt aatgtctcaa tgtattgcta gatacggagc cgaattttgt     600
   caacggttag cgagattctg ttatgcaatg aacagtttac aatatccagg agagactttt     660
   gatccacagc aacagactcc aacacaagta gcaagatgca tgaagtcaga aatggatagt     720
   ccgtccgtaa tgtggcactg tgtgcagaaa tatggtcaag aattttgtaa caagttagct     780
   gccacctgtt cgacagaaac gaacacacct cttccgcaac aggatccttg gcgaagctta     840
   attagc                                                                846
<210> 6
   <211> 282
   <212> PRT
   <213> Anisakis spp.
<220>
   <221> Peptide
   <222> (1)..(282)
   <223> Aminoacidic sequence of SEQ ID 5
<400> 6
   Met His Lys Cys Leu Lys Ser Glu Met Glu Asn Pro Gly Ile Met Ser
   1               5                   10                  15
   Thr Cys Val Gln Arg Tyr Gly Thr Gln Phe Cys Lys Asn Leu Leu Asn
               20                  25                  30
   Ser Cys Thr Ala Ser Thr Gly Met His Leu Pro Tyr Asp Ala Pro Trp
           35                  40                  45
   Lys Ile Pro Gln Pro Ile Ala Ala Cys Met Ala Gln Gly Met Asn Gly
       50                  55                  60
   Gly Asp His Lys Pro Lys Glu Asp Ala Met Ala Arg Cys Ile Arg Lys
   65                  70                  75                  80
   Tyr Gly Val Thr Tyr Cys Asn Asn Met Leu Ala Ser Cys Ser Val Leu
                   85                  90                  95
   Thr Asn Val Asn Tyr Asp Pro Ser Gly Gly Gln Met Pro Gly Ile Leu
               100                 105                 110
   Ser Glu Cys Val Thr Ala Glu Thr Asp Glu Pro Ser Ala Met Cys Gln
           115                 120                 125
   Cys Val Gln Lys Tyr Gly Thr Glu Phe Cys Asn Lys Arg Leu Ala Ser
       130                 135                 140
   Cys Ile Ala Ser Thr Gly Met Asn Leu Pro Ala Thr Thr Pro Trp Lys
   145                 150                 155                 160
   Leu Pro Pro Pro Val Ala Arg Cys Met Gln His Gly Ser Pro Asn Asp
                   165                 170                 175
   Asn Arg Gly Gln Gly Gln Gly Ser Asn Asp Val Met Ser Gln Cys Ile
               180                 185                 190
   Ala Arg Tyr Gly Ala Glu Phe Cys Gln Arg Leu Ala Arg Phe Cys Tyr
           195                 200                 205
   Ala Met Asn Ser Leu Gln Tyr Pro Gly Glu Thr Phe Asp Pro Gln Gln
       210                 215                 220
   Gln Thr Pro Thr Gln Val Ala Arg Cys Met Lys Ser Glu Met Asp Ser
   225                 230                 235                 240
   Pro Ser Val Met Trp His Cys Val Gln Lys Tyr Gly Gln Glu Phe Cys
                   245                 250                 255
   Asn Lys Leu Ala Ala Thr Cys Ser Thr Glu Thr Asn Thr Pro Leu Pro
               260                 265                 270
   Gln Gln Asp Pro Trp Arg Ser Leu Ile Ser
           275                 280
<210> 7
   <211> 12
   <212> PRT
   <213> Anisakis
<220>
   <221> Peptide
   <222> (1)..(12)
   <223> Antigenic Peptide
<400> 7
   Cys Val Gln Lys Tyr Gly Thr Glu Phe Cys Asn Lys
   1               5                   10
<210> 8
   <211> 12
   <212> PRT
   <213> Anisakis spp.
<220>
   <221> Peptide
   <222> (1)..(12)
   <223> Antigenic Peptide
<400> 8
   Cys Val Gln Lys Tyr Gly Gln Glu Phe Cys Asn Lys
   1               5                   10
<210> 9
   <211> 12
   <212> PRT
   <213> Anisakis spp.
<220>
   <221> Peptide
   <222> (1)..(12)
   <223> Antigenic Peptide
<400> 9
   Cys Val Gln Arg Tyr Gly Thr Gln Phe Cys Lys Asn
   1               5                   10
<210> 10
   <211> 12
   <212> PRT
   <213> Anisakis spp.
<220>
   <221> Peptide
   <222> (1)..(12)
   <223> Antigenic Peptide of Anisakis
<400> 10
   Cys Ile Arg Lys Tyr Gly Val Thr Tyr Cys Asn Asn
   1               5                   10
<210> 11
   <211> 12
   <212> PRT
   <213> Anisakis spp.
<220>
   <221> Peptide
   <222>
   <223> Antigenic Peptide
<400> 11
   Cys Ile Ala Arg Tyr Gly Ala Glu Phe Cys Gln Arg
   1               5                   10
<210> 12
   <211> 13
   <212> PRT
   <213> Anisakis spp.
<220>
   <221> Peptide
   <222> (1)..(13)
   <223> Antigenic Peptide
<400> 12
   Cys Leu Gln Lys Tyr Gly Ala Glu Phe Cys Asn Lys Leu
   1               5                   10
<210> 13
   <211> 11
   <212> PRT
   <213> Anisakis spp.
<220>
   <221> Peptide
   <222> (1)..(11)
   <223> Antigenic Peptide
<400> 13
   Lys Tyr Gly Glu Gln Phe Cys Ser Ser Met Leu
   1               5                   10
<210> 14
   <211> 9
   <212> PRT
   <213> Anisakis s
<220>
   <221> Peptide
   <222> (1)..(9)
   <223> Antigenic Peptide
<400> 14
   Cys Asn Arg Leu Gly Ala Ala Cys Ser
   1               5
<210> 15
   <211> 838
   <212> DNA
   <213> Anisakis spp.
<220>
   <221> misc_feature
   <222> (1)..(838)
   <223> Nucleotidic fragment of SEQ ID 3 which encodes for the antigenic sequence SEQ ID 16
<400> 15
   atgatgcaca aatgcttaaa gtcggaaatg gaaaatcctg gtattatgtc cacatgtgtt     60
   caaagatatg gtactcagtt ttgcaagaac ttactgaact cgtgcacagc gtcaaccggt    120
   atgcatcttc cgtacgatgc accttggaaa atacccgagc caatcgctgc ttgcatggcc    180
   caaggaatga atggtggtga ccacaaacct aaggaggatg cgatggcgag atgcatacgt    240
   aaatatggtg tcacttactg caacaacatg ctagcatctt gttctgtatt gaccaatgta    300
   aattatgacc ctagtggcgg tcaaatgccg ggaatattat cggaatgcgt gactgcagaa    360
   acggatgaac cgagtgcaat gtgccaatgc gttcaaaagt acggtactga attctgcaag    420
   aaacgccttg catcgtgtat tgcttctacg ggtatgaatc ttcccgccac tacaccatgg    480
   aaactgcctc cacctgtggc tcgctgcatg cagcacgggt cacctaatga caaccgcgga    540
   cagggacagg gttcaaatga cgtaatgtct caatgtattg ctagatacgg agccgaattt    600
   tgtcaacggt tagcgagatt ctgttatgca atgaacagtt tacaatatcc aggagagact    660
   tttgatccac agcaacagac tccaacacaa gtagcaagat gcatgaagtc agaaatggat    720
   agtccgtccg taatgtggca ctgtgtgcag aaatatggtc aagaattttg taacaagtta    780
   gctgccacct gttcgacaga aacgaacaca cctcttccgc aacaggatcc ttggcgaa      838
<210> 16
   <211> 279
   <212> PRT
   <213> Anisakis spp.
<220>
   <221> Peptide
   <222> (1)..(279)
   <223> Antigenic aminoacidic sequence
<400> 16
   Met Met His Lys Cys Leu Lys Ser Glu Met Glu Asn Pro Gly Ile Met
   1               5                   10                  15
   Ser Thr Cys Val Gln Arg Tyr Gly Thr Gln Phe Cys Lys Asn Leu Leu
               20                  25                  30
   Asn Ser Cys Thr Ala Ser Thr Gly Met His Leu Pro Tyr Asp Ala Pro
           35                  40                  45
   Trp Lys Ile Pro Gln Pro Ile Ala Ala Cys Met Ala Gln Gly Met Asn
       50                  55                  60
   Gly Gly Asp His Lys Pro Lys Glu Asp Ala Met Ala Arg Cys Ile Arg
   65                  70                  75                  80
   Lys Tyr Gly Val Thr Tyr Cys Asn Asn Met Leu Ala Ser Cys Ser Val
                   85                  90                  95
   Leu Thr Asn Val Asn Tyr Asp Pro Ser Gly Gly Gln Met Pro Gly Ile
               100                 105                 110
   Leu Ser Glu Cys Val Thr Ala Glu Thr Asp Glu Pro Ser Ala Met Cys
           115                 120                 125
   Gln Cys Val Gln Lys Tyr Gly Thr Glu Phe Cys Asn Lys Arg Leu Ala
       130                 135                 140
   Ser Cys Ile Ala Ser Thr Gly Met Asn Leu Pro Ala Thr Thr Pro Trp
   145                 150                 155                 160
   Lys Leu Pro Pro Pro Val Ala Arg Cys Met Gln His Gly Ser Pro Asn
                   165                 170                 175
   Asp Asn Arg Gly Gln Gly Gln Gly Ser Asn Asp Val Met Ser Gln Cys
               180                 185                 190
   Ile Ala Arg Tyr Gly Ala Glu Phe Cys Gln Arg Leu Ala Arg Phe Cys
           195                 200                 205
   Tyr Ala Met Asn Ser Leu Gln Tyr Pro Gly Glu Thr Phe Asp Pro Gln
       210                 215                 220
   Gln Gln Thr Pro Thr Gln Val Ala Arg Cys Met Lys Ser Glu Met Asp
   225                 230                 235                 240
   Ser Pro Ser Val Met Trp His Cys Val Gln Lys Tyr Gly Gln Glu Phe
                   245                 250                 255
   Cys Asn Lys Leu Ala Ala Thr Cys Ser Thr Glu Thr Asn Thr Pro Leu
               260                 265                 270
   Pro Gln Gln Asp Pro Trp Arg
           275
<210> 17
   <211> 834
   <212> DNA
   <213> Anisakis spp.
<220>
   <221> misc_feature
   <222> (1)..(834)
   <223> Nucleotidic fragment of SEQ ID N° 5 which encodes for the antigenic sequence SEQ ID N° 18
<400> 17
   atgcacaaat gcttaaagtc ggaaatggaa aatcctggta ttatgtccac atgtgttcaa     60
   agatatggta ctcagttttg caagaactta ctgaactcgt gcacagcgtc aaccggtatg    120
   catcttccgt acgatgcacc ttggaaaata cccgagccaa tcgctgcttg catggcccaa    180
   ggaatgaatg gtggtgacca caaacctaag gaggatgcga tggcgagatg catacgtaaa    240
   tatggtgtca cttactgcaa caacatgcta gcatcttgtt ctgtattgac caatgtaaat    300
   tatgacccta gtggcggtca aatgccggga atattatcgg aatgcgtgac tgcagaaacg    360
   gatgaaccga gtgcaatgtg ccaatgcgtt caaaagtacg gtactgaatt ctgcaagaaa    420
   cgccttgcat cgtgtattgc ttctacgggt atgaatcttc ccgccactac accatggaaa    480
   ctgcctccac ctgtggctcg ctgcatgcag cacgggtcac ctaatgacaa ccgcggacag    540
   ggacagggtt caaatgacgt aatgtctcaa tgtattgcta gatacggagc cgaattttgt    600
   caacggttag cgagattctg ttatgcaatg aacagtttac aatatccagg agagactttt    660
   gatccacagc aacagactcc aacacaagta gcaagatgca tgaagtcaga aatggatagt    720
   ccgtccgtaa tgtggcactg tgtgcagaaa tatggtcaag aattttgtaa caagttagct    780
   gccacctgtt cgacagaaac gaacacacct cttccgcaac aggatccttg gcga          834
<210> 18
   <211> 278
   <212> PRT
   <213> Anisakis spp.
<220>
   <221> PEPTIDE
   <222> (1)..(278)
   <223> Aminoacidic antigenic sequence
<400> 18
   Met His Lys Cys Leu Lys Ser Glu Met Glu Asn Pro Gly Ile Met Ser
   1               5                   10                  15
   Thr Cys Val Gln Arg Tyr Gly Thr Gln Phe Cys Lys Asn Leu Leu Asn
               20                  25                  30
   Ser Cys Thr Ala Ser Thr Gly Met His Leu Pro Tyr Asp Ala Pro Trp
           35                  40                  45
   Lys Ile Pro Gln Pro Ile Ala Ala Cys Met Ala Gln Gly Met Asn Gly
       50                  55                  60
   Gly Asp His Lys Pro Lys Glu Asp Ala Met Ala Arg Cys Ile Arg Lys
   65                  70                  75                  80
   Tyr Gly Val Thr Tyr Cys Asn Asn Met Leu Ala Ser Cys Ser Val Leu
                   85                  90                  95
   Thr Asn Val Asn Tyr Asp Pro Ser Gly Gly Gln Met Pro Gly Ile Leu
               100                 105                 110
   Ser Glu Cys Val Thr Ala Glu Thr Asp Glu Pro Ser Ala Met Cys Gln
           115                 120                 125
   Cys Val Gln Lys Tyr Gly Thr Glu Phe Cys Asn Lys Arg Leu Ala Ser
       130                 135                 140
   Cys Ile Ala Ser Thr Gly Met Asn Leu Pro Ala Thr Thr Pro Trp Lys
   145                 150                 155                 160
   Leu Pro Pro Pro Val Ala Arg Cys Met Gln His Gly Ser Pro Asn Asp
                   165                 170                 175
   Asn Arg Gly Gln Gly Gln Gly Ser Asn Asp Val Met Ser Gln Cys Ile
               180                 185                 190
   Ala Arg Tyr Gly Ala Glu Phe Cys Gln Arg Leu Ala Arg Phe Cys Tyr
           195                 200                 205
   Ala Met Asn Ser Leu Gln Tyr Pro Gly Glu Thr Phe Asp Pro Gln Gln
       210                 215                 220
   Gln Thr Pro Thr Gln Val Ala Arg Cys Met Lys Ser Glu Met Asp Ser
   225                 230                 235                 240
   Pro Ser Val Met Trp His Cys Val Gln Lys Tyr Gly Gln Glu Phe Cys
                   245                 250                 255
   Asn Lys Leu Ala Ala Thr Cys Ser Thr Glu Thr Asn Thr Pro Leu Pro
               260                 265                 270
   Gln Gln Asp Pro Trp Arg
           275
<210> 19
   <211> 20
   <212> DNA
   <213> Artificial sequence
<220>
   <223> Primer
<220>
   <221> Primer
   <222> (1)..(20)
<400> 19
   ttgtaaaacg acggccagtg 20
<210> 20
   <211> 20
   <212> DNA
   <213> Artificial sequence
<220>
   <223> Primer
<220>
   <221> Primer
   <222> (1)..(20)
<400> 20
   cctttgtcga tactggtact 20
<210> 21
   <211> 37
   <212> DNA
   <213> Artificial sequence
<220>
   <223> Primer
<400> 21
   gagagagagt cgacatgatg cacaaatgct taaagtc 37
<210> 22
   <211> 36
   <212> DNA
   <213> Artificial sequence
<220>
   <223> Primer
<220>
   <221> Primer
   <222> (1)..(36)
<400> 22
   gagagagaaa gctttcgcca aggatcctgt tgcgga 36
<210> 23
   <211> 22
   <212> PRT
   <213> Anisakis spp.
<220>
   <221> PEPTIDE
   <222> (1)..(22)
<400> 23
   Met Arg Gly Ser His His His His His His Thr Asp Pro His Ala Ser
   1               5                   10                  15
   Ser Val Pro Arg Val Asp
               20
<210> 24
   <211> 4
   <212> PRT
   <213> Anisakis spp.
<220>
   <221> PEPTIDE
   <222> (1)..(4)
<400> 24
   Ser Leu Ile Ser
   1
<210> 25
   <211> 21
   <212> DNA
   <213> Artificial sequence
<220>
   <223> Primer
<220>
   <221> Primer
   <222> (1)..(21)
<400> 25
   tgcacaaatg cttaaagtcg g 21
<210> 26
   <211> 20
   <212> DNA
   <213> Artificial sequence
<220>
   <223> Primer
<220>
   <221> Primer
   <222> (1)..(20)
<400> 26
   cagctaatta agcttcgcca 20
<210> 27
   <211> 20
   <212> DNA
   <213> Artificial sequence
<220>
   <223> Primer
<220>
   <221> Primer
   <222> (1)..(20)
<400> 27
   tgaatggtgg tgaccacaaa 20
<210> 28
   <211> 21
   <212> DNA
   <213> Artificial sequence
<220>
   <223> Primer
<220>
   <221> Primer
   <222> (1)..(21)
<400> 28
   tggatcaaaa gtctctcctg g 21

## Description

This invention relates to the field of diagnosis, prevention and therapy of human and animal infections by species of the genus *Anisakis.* This invention also relates to the detection of antigens of *Anisakis* in foods.

### BACKGROUND OF THE INVENTION

The genus *Anisakis* (family Anisakidae) includes nematode parasites that belong to several related species. Using morphological and genetic/molecular markers, currently 8 species of *Anisakis* are recognised, which include: a) the sister species *Anisakis simplex* "strictu sensu" (Rudolphi, 1809), *A. pegreffi* (Campana-Rouget and Biocca, 1955) and *A. simplex* C (Nascetti et al., 1986, Mattiucci et al., 2002), formerly included in the *A. simplex* complex; b) *A. typica* (Diesing, 1860); c) *A. physeteris* (Baylis, 1923); d) *A. brevispiculata* (Dollfus, 1966); e) *A. ziphidarum* (Paggi et al., 1998); and f) *A. paggiae* (Mattiucci et al. 2005).

*Anisakis* species reach sexual maturity in the stomach of cetaceans and, less frequently, in pinnipeds. These mammals may become infected by the ingestion of a) paratenic hosts, i.e. fish (mainly teleosteans) and cephalopods (mainly squid), which host the parasite's third larva phase (L3), and b) by the ingestion of krill, which also hosts the L3 larvae, as an intermediate (for example, whales).

As is the case with marine mammals, human beings may also become infected with L3 *Anisakis* larvae by the ingestion of raw fish and squid that carry the parasite, which causes a clinical disease called anisakiosis or anisakiasis. Frequently, several fish dishes are considered to entail a high risk of infection with Anisakis species. These include Japanese sushi and sashimi, German sauteed or smoked herring, Scandinavian gravlax, Hawaiian lomi-lomi, South American ceviche and Spanish pickled anchovies.

When the L3 *Anisakis* larvae infect human beings, they often cause gastrointestinal symptoms that may be associated with allergic reactions of variable severity. Depending on the location of the larvae and the dominant symptoms, four main clinical forms of anisakiosis (gastric/gastroallergic, intestinal, extragastrointestinal and allergic) have appeared in human beings. In the gastric (acute) form of anisakiosis, the larva penetrates the gastric wall and frequently a clinical course is observed characterised by acute epigastric pain, nausea and vomiting a few hours after the ingestion of fish containing live L3 *Anisakis* larvae. Endoscopic studies frequently reveal lesions consisting of punctate haemorrhages, petechiae and erosion at the penetration site. Moreover, allergic symptoms (i.e. urticaria/angioedema and sometimes anaphylaxis) may also be observed in approximately 10% of the cases. The combination of gastric infection and allergic symptoms was called "gastroallergic anisakiosis" (Daschner et al., 2000). Some patients may develop a subacute or chronic form, the clinical manifestations whereof include epigastric pain, dyspepsia, vomiting and anorexia, which may persist for several months or even years.

In the intestinal form of anisakiosis, the larva penetrates the intestinal wall, frequently at the level of the terminal ileon. In acute cases, the presence of abdominal pain is often observed (usually 24-48 hours after the ingestion of the contaminated fish), accompanied by nausea, vomiting, abdominal swelling, induration and deviation of the established intestinal rhythm with constipation or diarrhoea. Macroscopic observation of the infected tissues, in the case of intestinal anisakiosis, reveals phlegmonous lesions characterised by intense local edema, petechiae, hyperemia and diffuse inflammation of the serous membrane and the mesenterium. These lesions may cause proximal obstruction and dilatation of the intestine. In a significant number of patients, abdominal ascitic fluid with a high content of eosinophils may be found. In subacute or chronic intestinal anisakiosis, the granulomatous changes induced by the larvae lead to a thickening of the wall, luminal stenosis and chronic abdominal symptoms.

In the extragastrointestinal or ectopic form, the infective *Anisakis* larva crosses the gastrointestinal wall, reaching the abdominal cavity and migrating to organs and tissues such as the lungs, pancreas, liver, etc. In these cases, the clinical symptoms depend on the larva's topographic location.

The allergic anisakiosis form is reserved for patients who have allergic reactions after the infection with *Anisakis* larvae, but no gastric or intestinal symptoms. Frequently, the first symptoms appear very soon, during the first few hours after the ingestion of the contaminated fish and the clinical course follows the general pattern for type I allergic reactions. The severity varies from a simple urticaria or angioedema to anaphylactic shock.

When the *Anisakis* larvae are present in the stomach, the duodenum or the colon, the preferred method to demonstrate the infection is endoscopic examination. This technique is also useful to remove the larvae by means of the pincers associated with the endoscope, which leads to healing of the patient. However, when: a) the larvae are not accessible for the endoscopic examination; b) the larvae are partially destroyed or are not visible to the endoscopist; or c) the predominant symptoms are allergic, the preferred method to confirm the infection is to demonstrate the presence of specific antibodies in the patient's serum. The latter is also the preferred method to demonstrate previous infections by *Anisakis* in asymptomatic subjects.

Amongst the different classes of antibodies against antigens of *Anisakis* that are produced during the infection, IgE antibodies are the most relevant, since: 1) they are involved in the type I allergic reactions observed during infections by *Anisakis,* and b) this is the most relevant class of immunoglobulins in terms of specificity amongst those present in the serum of infected patients.

Since the description of the first case of human infection by an *Anisakis* larva (Van Thiel, 1960), numerous attempts have been made to develop a sensitive, specific serological assay to detect different classes of *anti-Anisakis* antibodies.

The first methods designed to measure anti-*Anisakis* antibodies in serum included latex agglutination (Akao and Yoshhimura, 1989); double immunodiffusion and immunoelectrophoresis (Petithory et al., 1986; Tsuji, 1990), indirect haemagglutination (Asaishi et al., 1989; Tsuji, 1989); and complement fixation assay (Oshima, 1972; Tsuji, 1989). All these methods have two major disadvantages: a) they lack sufficient sensitivity and specificity, and b) it is not possible to determine IgE antibodies (reagins) using these techniques.

More recently, determinations of anti-*Anisakis* IgE in the sera of infected patients were made thanks to the implementation of other immunoassays, for example, radioallergoabsorbent tests (Desowitz et al., 1985; Yamamoto et al., 1990), enzyme-linked immunoabsorption assays (ELISA) (Rodero et al. 2002), fluorescent enzymoimmunoassays (UniCap FEIA, Pharmacia & Upjohn Diagnostics AB, Uppsala, Sweden) and immunotransfer (Del Pozo et al., 1996; Garcia et al., 1997), but sensitivity and/or specificity problems also arose with these methods.

The main difficulty in the development of useful serological methods for the diagnosis of human *Anisakiosis* lies in obtaining parasite antigens (in our case allergens) which, on the one hand, (i) are recognised by the IgE antibodies present in the sera of all infected patients, and (ii) that the complete molecule (i.e. all the epitopes present in the selected allergen) is specific for the genus *Anisakis.* Otherwise, a low sensitivity and/or cross-reactions leading to false positives would be obtained.

There was an attempt to improve the serodiagnosis of human anisakiosis using complete native excretion/secretion antigens (ESA) of *Anisakis* spp. larvae cultured *in vitro* (Poggensee et al., 1989; Baeza et al., 2004), or using monoclonal antibodies to capture specific target antigens from crude extracts of L3 *Anisakis* larvae (Yagihashi et al., 1990; Ubeira and Iglesias, 2000). However, the ELISA methods based on these strategies also lacked sufficient specificity due to the presence of cross-reaction epitopes (primarily *O*- and *N*-glycans) present in the glycoproteins of *Anisakis,* including ESA.

### DESCRIPTION OF THE INVENTION

This invention provides peptide, polypeptide and protein sequences that maintain the antigenicity of the native molecules of *Anisakis* wherefrom they derive, as well as the nucleic acids that encode said sequences and fragments thereof for said peptides, polypeptides or proteins. The peptides, polypeptides and proteins disclosed, as well as the fragments thereof against said peptides, polypeptides and proteins are useful to develop immunoassays for the serodiagnosis of human and animal anisakiosis, and lack the disadvantages of the methods described above.

Therefore, a first aspect of this invention provides an isolated nucleic acid sequence (hereinafter called nucleic acid sequence of the invention) selected from the following group:
a. A nucleic acid that comprises SEQ ID No. 1.
b. A nucleic acid that comprises the complementary nucleic acid of SEQ ID No. 1.
c. A fragment of the nucleic acid of SEQ ID No. 1 that comprises SEQ ID No. 15 or 17.
d. A nucleic acid that comprises a fragment of SEQ ID No. 1 which encodes a stretch of 10 or more adjacent amino acids of SEQ ID No. 2 capable of being recognised by anti-*Anisakis* antibodies.
e. A nucleic acid that comprises a fragment of SEQ ID No. 1 which encodes a stretch of 12 or more adjacent amino acids of SEQ ID No. 2 capable of being recognised by anti-*Anisakis* antibodies.

A second aspect of this invention provides an isolated polypeptide, peptide or protein (hereinafter called amino acid sequence of the invention) selected from the following group:
a. A polypeptide that comprises SEQ ID No. 2.
b. A fragment of SEQ ID No. 2 that comprises SEQ ID No. 16 or 18.
c. A fragment of SEQ ID No. 2 that comprises a stretch of 10 or more adjacent amino acids capable of being recognised by anti-*Anisakis* antibodies.
d. A fragment of SEQ ID No. 2 that comprises a stretch of 12 or more adjacent amino acids capable of being recognised by anti-*Anisakis* antibodies.
e. An amino acid sequence that has an identity of at least 70% with the amino acid sequences described in section (b) of this item. In a preferred embodiment of the invention, the amino acid sequence has an identity of at least 80% with the amino acid sequences previously described in sections (b) of this item and, in an even more preferred embodiment, said amino acid sequence has an identity of at least 90% with the amino acid sequences previously described in section (b) or (d) of this item.
All these peptides, polypeptides or proteins may be used to overcome the main difficulty in developing useful serological methods for the diagnosis of human anisakiosis, by providing specific antigens of the *Anisakis* genus parasite which are recognised by the IgE antibodies present in the sera of all patients infected by *Anisakis.*

A preferred embodiment of the invention relates to an amino acid sequence that may be recognised by *anti-Anisakis* antibodies, which comprises an amino acid sequence, selected from the group:
a. An amino acid sequence that comprises SEQ ID No. 7.
b. An amino acid sequence that comprises SEQ ID No. 8.
c. An amino acid sequence that comprises SEQ ID No. 9.
d. An amino acid sequence that comprises SEQ ID No. 10.
e. An amino acid sequence that comprises SEQ ID No. 11.
f. An amino acid sequence that comprises SEQ ID No. 12.
g. An amino acid sequence that comprises SEQ ID No. 13.
h. An amino acid sequence that comprises SEQ ID No. 14.

In a third aspect, the amino acid sequence of the invention is produced by any of the following techniques, but without being limited thereto:
a. Recombinant techniques
b. Chemical synthesis and
c. Substantial purification from nematodes of the family Anisakidae

A fourth aspect of the invention relates to a peptide, polypeptide or protein that comprises two or more of the amino acid sequences of the invention.

A fifth aspect of the invention relates to a synthetic or recombinant nucleic acid molecule that comprises a nucleic acid sequence that encodes one or more of the amino acid sequences of the invention.

A sixth aspect of the invention relates to an isolated nucleic acid or amino acid sequence of the invention, labelled with a signalling molecule. In a preferred embodiment of the invention, said labelling molecule is selected from any of the signalling molecules of the following group, but without being limited thereto:
a. Radioactive
b. Enzymatic
c. Fluorescent
d. Luminescent
e. Chemiluminescent
f. Coloured

In a preferred embodiment of the invention, the labelling molecule is selected from any of the signalling molecules of the following group, but without being limited thereto:
a. Biotin or the derivatives thereof
b. Nitrophenol derivatives
c. Colloidal gold
d. Latex

Another aspect of the invention provides an isolated amino acid sequence of the invention, where said amino acid sequence is chemically bound or coupled to an additional peptide, polypeptide or protein.

Another aspect of the invention relates to an expression vector or expression system (hereinafter called expression system of the invention) that comprises a nucleotide sequence of the invention.

Another aspect of the invention provides an isolated prokaryotic or eukaryotic host cell (hereinafter called host cell of the invention) transformed or transfected with a nucleic acid sequence of the invention or with an expression vector or system of the invention.

An eighth aspect of the invention relates to a method of producing a recombinant peptide, polypeptide or protein encoded by a nucleic acid sequence of the invention, where said method comprises culturing a host cell of the invention under such conditions that said nucleotide sequence is expressed and produced, and subsequently isolating said peptide, polypeptide or protein.

A ninth aspect of the invention relates to an *in vitro* method of detecting antibodies against *Anisakis* spp. in an isolated biological sample, preferably serum, which comprises:
a. placing a biological sample in contact with an amino acid sequence of the invention, under sufficient conditions to form an immunological complex between said polypeptide and the antibodies in the sample, and
b. detecting said immunological complex.

In a preferred embodiment of the invention, the *in vitro* method is an immunoassay or an enzymatic immunoassay.

A preferred aspect of the invention relates to an *in vivo* method of diagnosing or prognosticating allergy to *Anisakis* spp. in a subject, which comprises exposing the subject to the peptide, polypeptide or protein of the invention and monitoring the subject's reaction.

An even more preferred aspect of the *in vivo* method of the invention of detecting antibodies against *Anisakis* spp. is using the *Prick* assay.

Another preferred aspect of this invention is a kit suitable to detect *anti-Anisakis* spp. antibodies in a biological sample, preferably serum, which comprises:
a) at least one peptide, polypeptide or protein of the invention and
b) a means of detection of the immunological complex.

In a preferred embodiment, the means of detection used in the kit of the invention consists of labelled secondary antibodies capable of recognising the formation of the immunological complex.

In an even more preferred embodiment, the means of detection used in the kit of the invention is a labelled secondary reagent capable of recognising the formation of the immunological complex.

Another aspect of the invention relates to the use of an amino acid sequence of the invention to manufacture a pharmaceutical composition for the treatment of any type of allergic reaction to *Anisakis* spp. A preferred embodiment of the invention relates to the pharmaceutical composition as such.

As used in this document, "nucleic acid" or "nucleic acid molecule" refers to any polymer of nucleotides composed of two or more subunits that are deoxyribonucleotides or ribonucleotides, bound to one another by phosphodiester bridges. "Nucleic acids" or "nucleic acid molecules" include deoxyribonucleic acid (DNA), ribonucleic acid (RNA), oligonucleotides and fragments generated by polymerase chain reaction (PCR) or by other methods such as linkage, splicing, endonuclease action or exonuclease action. As used in this document, the term "nucleotide" means a monomer unit of DNA or RNA that contains a remainder of sugar (pentose), a phosphate and a nitrogenated heterocyclic base. The four bases of DNA are adenine ("A"), guanine ("G"), cytosine ("C") and thymine ("T"). The four bases of RNA are A, G, C and uracil ("U").

As used in this document, an "isolated nucleic acid molecule" is a nucleic acid molecule that is not integrated in an organism's genomic DNA. Said nucleic acid molecule may be separated from a cell's genomic DNA, produced using recombinant DNA technology (for example, PCR amplification, cloning, etc.) or chemically synthesised. The isolated nucleic acid molecule may be obtained from its natural source as a complete gene or a part thereof capable of forming a stable hybrid with that gene. The nucleic acid molecule may be single-chain or double-chain.

As used in this document, "primer" refers to a natural or synthetic oligonucleotide (which preferably ranges from 15 to 40 nucleotides in length) that may hybridise with a complementary DNA or RNA template to form a primer-template duplex. Typically, a primer is a single-chain oligodeoxyribonucleotide, and acts as the starting point for the synthesis of nucleic acid by a polymerase following hybridisation with a DNA or RNA chain.

As used in this document, the expression "nucleotide sequence that encodes" refers to a nucleic acid sequence that directs the expression of a specific peptide, polypeptide or protein. The nucleic acid sequences comprise the DNA chain sequence that is transcribed in RNA and also the RNA sequence that is translated into a protein. The nucleic acid sequences of this invention include both full-length nucleic acid sequences and truncated sequences (those that do not have the full length) derived from the full-length protein. They also comprise the variants of the nucleotide sequence that encode the same peptide, polypeptide or protein as the native sequence, which may be constructed to provide a codon preference in a specific host cell.

As used in this document, "the complement of" refers to the concept of an inverse correspondence between regions of two polynucleotide chains or between two nucleotides through the formation of base pairs. Consequently, a "sequence of complementary bases" refers to a polynucleotide chain wherein all the bases may form base pairs with a sequence of bases in another polynucleotide chain. As is well known, an adenine nucleotide may form base pairs with thymine (A-T) or uracil (A-U), whereas a cytosine nucleotide may form base pairs with guanine (C-G).

As used in this document, an "expression vector" refers to an assembly that is capable of directing the expression of a sequence or gene of interest. According to this invention, the expression vector may be any DNA or RNA expression vector capable of expressing antigens of *Anisakis* spp., such as a plasmid or a phage. Preferably, the expression vector in accordance with this invention is a plasmid (for example, pQE 31 and pTARGET).

As used in this document, the term "plasmid" refers to any autonomous circular DNA molecule capable of replicating in a cell independently from the chromosomal DNA, and includes both expression and non-expression types.

As used in this document, the term "recombinant" refers to a compound DNA molecule prepared outside live cells by artificially binding natural or synthetic DNA fragments to DNA molecules that may replicate in a live cell, or molecules that are the result of the replication thereof. In this document, the term "recombinant" also refers to a peptide, polypeptide or protein that is expressed using a recombinant DNA molecule.

The terms "polypeptide" and "protein" are used interchangeably in this document to designate a linear sequence of amino acids bound to one another by amide bonds between the alpha-amino group of one amino acid and an alpha-carboxyl group of the adjacent amino acid. Polypeptides that have a length of twenty-five amino acids or less are considered to be "peptides". Preferably, a polypeptide that is encoded by a cistron is considered to be a "protein".

As used in this document, the expression "fragment of the amino acid sequence" means a portion of adjacent amino acids in the specified peptide, polypeptide, protein or amino acid sequence.

As used in this document, an "isolated peptide, polypeptide or protein" is a peptide, polypeptide or protein that is essentially free of cell contaminants, such as lipids, carbohydrates or other impurities associated with the polypeptide in nature. An isolated peptide, polypeptide or protein may be purified from its natural medium by conventional chromatographic techniques. Isolated peptides, polypeptides and proteins may also be obtained by recombinant methods, or by chemical synthesis. Typically, a "substantially purified polypeptide" is obtained when it is separated from at least 50%, more preferably at least 75%, and even more preferably at least 90%, of the other polypeptides wherewith it naturally co-exists in a cell, tissue or organism.

As used in this document, the terms "amino acid" and "amino acids" refer to all the L-alpha-amino acids of natural origin or the remainders thereof. The following Table (Table 1) shows the three-letter code and the one-letter code (recommended by the IUPAC-IUB Commission on Biochemical Nomenclature) used to designate natural amino acids:

| | | | | | |
|---|---|---|---|---|---|
| Asp | D | aspartic acid | Ile | I | isoleucine |
| Thr | T | threonine | Leu | L | leucine |
| Ser | S | serine | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |
| Val | V | valine | Gln | Q | glutamine |
| Met | M | methionine | Asn | N | asparagine |

As used in this document, the term "mammal" refers to any animal classified as a mammal, including human beings, mice, rats, marine mammals and domestic and farm animals. Preferably, the mammal in this document is a human being.

As used in this document, the term "antibody" refers to glycoproteins of the immunoglobulin family, characterised in that they have antigen-binding properties and are produced by plasma cells. The term "antibody" includes both monoclonal and polyclonal antibodies belonging to any class of antibodies, for example, IgG, IgD, IgM, IgA, IgE or derivatives thereof.

As used in this document, the expression "monoclonal antibody" refers to a composition of antibodies with a homogeneous population of antibodies. It is not intended to be limited to a particular source of antibodies or to the manner wherein it is produced, and includes antibodies and fragments thereof produced by cell fusion or by recombinant techniques.

As used in this document, the term "antigen" refers to any molecule or agent capable of inducing the production of specific antibodies when it is introduced into an immunocompetent host. As used in this document, the term "antigen" also refers to any molecule or agent susceptible of being recognised by a specific antibody. Typically, peptides, polypeptides, proteins, carbohydrates, lipids, nucleic acids or combinations of these molecules may be recognised as antigens. These antigens may also contain bound organic and inorganic chemical groups.

As used in this document, the term "epitope" refers to the portion of an antigen that is recognised by a (monoclonal or polyclonal) antibody or by a cell's antigen-specific receptor. When the antigen is a peptide, a polypeptide or a protein, the epitopes may be fragments of at least 5 amino acids of the primary amino acid sequence, but also exposed regions on the surface of the mature folded protein (tertiary three-dimensional structure) composed of 5 or more amino acids. Typically, epitopes may be classified as B-cell epitopes and T-cell epitopes on the basis of the types of immune response which they cause.

As used in this document, the term "biological sample" is applied to any sample of biological tissue or fluid that contains one or more of the nucleic acids, antibodies, peptides, polypeptides or proteins of this invention. In this invention, the term "biological sample" is also applied to any sample of biological tissue or fluid that may be used as a source to determine anti-*Anisakis* antibodies. Said samples include, but are not limited thereto, isolated tissues, blood, serum, plasma, cerebrospinal fluid, ascitic fluid, sinovial fluid, saliva, urine or faeces. The biological samples may also include tissue sections such as frozen sections taken for histological purposes. A biological sample may be obtained from any eukaryotic organism, preferably from a mammal, such as a rat, mouse, rabbit, marine mammal or human being.

As used in this document, the term "food sample" refers to any sample obtained from the broad range of edible materials. These samples include, but are not limited thereto, pieces of raw, smoked and cooked fish, and foods that contain fish materials in their composition. The term "food sample" also includes baby nutrition foods.

As used in this document, the expression "anti-*Anisakis* antibodies" refers to any class or subclass of antibodies present in a sample capable of recognising at least one epitope that is present in the antigens of any species of the genus *Anisakis.* The expression includes antibodies produced during natural or experimental infections of human beings and animals with the parasite, as well as monoclonal and polyclonal antibodies produced after the immunisation of human beings or animals with non-purified or substantially purified recombinant or synthetic antigens, obtained from species of the genus *Anisakis.* The expression also includes antigen-binding antibodies and fragments produced by recombinant techniques, chemical synthesis or equivalent techniques.

As used in this document, the expression "percentage of identity" refers to the percentage of amino acids or nucleotides that occupy the same relative position when two amino acid sequences, or two nucleic acid sequences, are aligned next to one another. For purposes of this invention, a suitable method to calculate the percentage of sequence identity between two nucleotide or amino acid sequences is William Pearson's *lalign* programme, which applies the Huang and Miller algorithm published in Adv. Appl. Math. (1991) 12: 337-357, which is incorporated into this document as a reference. This programme may be processed online at: [http://www.ch.embnet.org/software/LALIGN_form.html].

As used in this document, the term "immunoassay" refers to a method of detecting an analyte in a sample by means of an antigen-antibody reaction. The analytes may be small molecules (haptens) or large molecules, such as many plasma proteins. Frequently, the analyte is an antibody that may be detected by the specific bond between the antibody and a ligand. As used in this document, the term "enzymatic immunoassay" (EIA) refers to a broad type of immunoassays, characterised in that at least one of the reagents is labelled with an enzyme. Typical EIAs include enzyme-linked immunoabsorption assays (ELISAs), which have a number of variants. For a classification or a detailed description of immunoassay formats, see: Price and Newman (1997). Principles and Practice of Immunoassay, Macmillan Reference LTD, New York; Tijssen (1985) Practice and theory of enzyme immunoassays, In: Laboratory Techniques in Biochemistry and Molecular Biology (R.H. Burdon and PH van Knippenberg, eds), Elsevier, Amsterdam; Deshpande (1996) Enzyme immunoassays, from concept to product development, Chapman & Hall, New York.

As used in this document, a "pharmaceutical composition" refers to a chemical or biological composition that is suitable to be administered to a mammalian subject. Typically, a pharmaceutical composition comprises a quantity of a pharmacologically effective active agent and a pharmaceutically acceptable carrier. Examples of suitable carriers and pharmaceutical formulations are described in Remington's Pharmaceutical Sciences, 20th edition, Mack Publishing Company, Easton, 2000. A pharmaceutical composition may be specifically formulated for administration by different routes, including, but not limited thereto, oral, parenteral, intravenous, intraarterial, subcutaneous, intranasal, sublingual and similar routes.

As used in this document, the expression "diagnostic method" refers to a method of identifying a pathological condition in an animal or a subject. When said pathological condition is produced by an infectious agent, the diagnosis may often be made using an immunoassay (see above) that allows to detect the antigens released by the pathogen, or the antibodies induced by said pathogen in the host. The diagnostic methods of this invention include *in vitro* assays (such as an ELISA assay, or the basophil histamine release test) and *in vivo* assays such as cutaneous tests (for example, but not limited thereto, the prick test), intradermoreactions or epicutaneous tests (for example, the patch test), which are frequently used to diagnose allergies. The expression "diagnostic methods" also includes provocation tests, which consist of the controlled, gradual administration of the substance suspected of causing allergic symptoms by different routes: oral, conjunctival, nasal, bronchial, etc., in order to check the tolerance thereof. A more detailed description of the latter methods may be found in: "Comité de Reacciones Adversas a Alimentos: Metodología diagnóstica en la alergia a alimentos". Alergología e Inmunología Clínica, 14: 50-52 (1999), as well as in various manuals in the Clinical Allergology and Immunology speciality.

As used in this document, the expression "sequence repetitions" refers to repetitive motifs in the sequence of a given protein or a nucleic acid molecule. The sequence repetitions in the polypeptide sequences of this invention were performed using the RADAR programme (see EXAMPLE No. 12).

Except as otherwise defined, the technical and scientific terms used in this document have the same meaning that is usually understood by a person skilled in the art whereto this invention belongs. In the practise of this invention, similar or equivalent methods and materials to those disclosed in this document may be used. Throughout the entire description and the claims, the word "comprises" and the variations thereof are not intended to exclude other technical characteristics, additives, components or steps. Other objects, advantages and characteristics of the invention will be evident for those skilled in the art after studying the description or may be learnt by the practise of the invention. The following examples are provided for illustrative purposes and are not intended to limit this invention.

### EXAMPLES

### EXAMPLE 1. Isolation of mRNA and construction of an expression library from Anisakis simplex.

*Anisakis simplex* larvae in the L3 larva phase (L3) were manually extracted from the viscerae and the peritoneal cavity of blue whiting (*Micromesistius poutassou*) and stored in liquid nitrogen prior to starting the construction of the cDNA library. The total messenger RNA (mRNA) was obtained from 1 g of frozen L3 *Anisakis simplex* larvae using the Fast Track 2.0 mRNA isolation kit (Invitrogen S.A., Barcelona, Spain) in accordance with the manufacturer's instructions.

Five micrograms of poly (A)+ RNA obtained to construct the *Anisakis* cDNA library using the ZAP-cDNA Gigapack III Gold Cloning Kit (Stratagene, La Jolla, CA) were used, following the manufacturer's instructions. In accordance with the instructions provided, the poly (A)+ RNA of *Anisakis simplex* was first converted into double-chain cDNA; subsequently, it was bound to the lambda-ZAP expression vector and the vector was packed using Gigapack III packing extracts. The XL1-Blue MRF' strain of *E. coli* was used as a host. The resulting library was amplified, and it contained approximately 1.3 x 10¹⁰ pfu/ml and 1% of non-recombinant phages.

### Exploration of the cDNA library of Anisakis simplex

After it was amplified, the cDNA library of *A*. *simplex* was subject to an immunological screening using UA3 monoclonal antibodies. In accordance with the recommendations provided in the ZAP-cDNA Gigapack III Gold Cloning Kit (Stratagene), XL1-Blue MRF' host cells were allowed to grow in supplemented LB broth, centrifuged at 1,000 x *g*, and the sediment was re-suspended in 10 mM magnesium sulfate. An aliquot of the recombinant phages was added to this suspension and the mixture was incubated for 15 minutes at 37ºC in order to allow the phages to bind to the cells. Subsequently, the cell suspension with the bound phages was mixed with melted NZY agar (cooled to approximately 55ºC), and the mixture was uniformly spread on a 150-mm diameter plate that contained newly poured NZY agar. Following an incubation time of approximately 3 h at 42ºC, to allow the phage plaques to begin to form, the expression of recombinant proteins was induced by coating the plates with nitrocellulose filters previously impregnated in 10 mM isopropyl thio-beta-D-galactoside (IPTG) in water (Schleicher & Schuell, Dassel, Germany). Subsequently, the plates were incubated at 37ºC for 3.5 h, and the nitrocellulose filters containing the adsorbed phage plaques were carefully removed and blocked with 3% BSA in TBS (50 mM Tris-HCl, 150 mM NaCl, pH 7.5) at 37ºC for 30 minutes and subsequently incubated with IgG1/kappa UA3 monoclonal antibodies (MAb) (dilution 1:10,000 in TBS) at 4ºC overnight. The nitrocellulose filters were washed in TBS containing 0.05% Tween 20 (TBS-T), and incubated for 2 h at ambient temperature with alkaline-phosphatase-conjugated goat anti-rabbit IgG at 1:10,000 (Pierce, Rockford, IL, USA). After washing once again, the immune complexes were viewed using NBT (nitroblue tetrazolium) and BCIP (*p*-toluidine-5-bromo-4-chloro-3-indolyl phosphate) (Sigma Chemical Corp., St Louis, MO, USA).

### In vivo splicing of a single clone and cDNA sequencing

After identifying a cDNA that encoded a protein recognised by MAb UA3 by means of immunological screening of the intact lambda-ZAP vector, the corresponding phage plaque (UAR-H5 clone) was purified. In accordance with the instructions provided in the ZAP-cDNA Gigapack III Gold Cloning Kit (Stratagene), the central portion of the positive plates selected for the phage was transferred to a microcentrifuge tube and the recombinant phage particles were released in SM buffer. Subsequently, *in vivo* splicing of the pBluescript phagemid of the lambda-ZAP vector was performed by co-infecting XL1-Blue MRF' host cells with the recombinant phage and the ExAssist auxiliary phage supplied with the kit. At the end of this procedure, the spliced pBluescript phagemid, which was obtained packed in the form of filamentous phage particles, was used to infect SOLR host cells. Colonies of transfected SOLR cells were obtained which contained the double-chain pBluescript phagemid with the cloned DNA insert of interest, and the cells were allowed to grow in Luria-Bertani (LB) agar plates containing ampicillin (100 micrograms/ml). Individual bacterial colonies were allowed to grow in LB broth-ampicillin and the pBluescript phagemid's DNA was purified using a Perfectprep Plasmid Maxi kit (Eppendorf). Direct (5' SEQ ID No. 19 3') and reverse (5' SEQ ID No. 20 3') M13 primers of the pBluescript SK sequence were used, jointly with several internal primers, for the automatic sequencing of the phagemid's DNA, using the Big-Dye Terminator Cycle Sequencing Ready Reaction Kit (PE Biosystems, Foster City, CA) and an Applied Biosystems 377 DNA sequencer (PE Biosystems). The DNA sequence and the deduced amino acid sequence were compared with the EMBL and SWISS-PROT data banks using the ExPASy software package (the Expert Protein Analysis System), World Wide Web server (http://www.expasy.org/), from the Swiss Institute of Bioinformatics (Gasteiger et al., 2003). The nucleotide sequence described comprises a sequence of 3,288 nucleotides, represented by the sequence of SEQ ID No.: 1, and encodes a deduced amino acid sequence of 1,096 amino acids represented by SEQ ID No.: 2.

### EXAMPLE 2. Subcloning of a fragment of the cDNA sequence (SEQ ID No.: 1) of Anisakis simplex in the pQE-31 vector.

In a preferred embodiment, a fragment of recombinant internal DNA (SEQ ID No.: 15), corresponding to the positions of nucleotides 1303 and 2139 of SEQ ID No. 1, which encodes a polypeptide sequence of 279 amino acids (SEQ ID No.: 16), was subcloned in expression vector pQE-31 using a QIAexpress Type IV kit (Qiagen, IZASA S.A., Barcelona, Spain) in accordance with the manufacturer's instructions, using restriction sites *Sal* I and *Hind* III at the multiple cloning site of pQE-31. Another similar methodology is available in manuals such as: Molecular Cloning: a Laboratory Manual (Sambrook et al., 2001) or Current Protocols in Molecular Biology (Ausubel et al., 1998).

Prior to the cloning, the sequence of the cDNA fragment corresponding to SEQ ID No.: 15 was amplified by PCR, and the ends of the sequence were modified using the recombinant pBluescript phagemid as a template and a set of direct (5' SEQ ID No. 21 3') and reverse primers (5' SEQ ID No. 22 3') containing restriction sites *Sal* I and *Hind* III. This PCR modification provided specific nucleotide sequences for restriction enzymes *Sal* I and *Hind* III in order for the recombinant insert to have cohesive ends with respect to the digested plasmid. The PCR conditions for the amplification and modification of the sequence of the DNA fragment corresponding to SEQ ID No.: 15 comprised: a denaturation cycle at 94ºC for 4 minutes followed by 35 cycles of (30 s of denaturation at 94ºC; 30 s of hybridisation at 55ºC; and one minute of elongation at 72ºC), and a final elongation cycle at 72ºC for 7 minutes. After the PCR was concluded, the amplified recombinant DNA of *Anisakis* was purified using a QiaQuick gel purification kit (Qiagen). Subsequently, one microgram of the plasmid DNA and 2 micrograms of the DNA that encoded the fragment to be inserted were digested with restriction enzymes *Sal* I and *Hind* III, in accordance with the buffer and the incubation conditions recommended by the manufacturer (Invitrogen S.A., Barcelona, Spain). Finally, both the digested vector and the DNA insert were purified with the QiaQuick gel purification kit (Qiagen) and ligated in a 1:3 proportion between the vector and the insert, using T4 DNA ligase at 16ºC overnight. The insertion of the *Anisakis simplex* recombinant DNA of SEQ ID No.: 15 between sites *Sal* I and *Hind* III of the multiple cloning site of pQE-31 led to a plasmid open reading frame (ORF) of 915 nucleotides (SEQ ID No.: 3) that encoded the amino acid sequence of SEQ ID No.: 4, which includes SEQ ID No.: 16 of *Anisakis* (central portion) and two additional amino acid sequences (tails): SEQ ID No. 23 and SEQ ID No. 24, located, respectively, at the terminal amino and carboxyl portions of the cloned *Anisakis* sequence. The presence of the 6 x Histidine signal in the amino prtion of the encoded polypeptide may facilitate the purification of recombinant polypeptides, but other vectors and purification methods may also be used, as convenient.

### EXAMPLE 3. Transformation of M15 cells (pREP4) with recombinant pQE-31

The transformation of M15 cells [pREP4] of *E. coli* with recombinant vector pQE-31 was performed in accordance with the instructions provided in the manufacturer's (Qiagen) manual (The QIAexpressionist: A handbook for high-level expression and purification of 6xHis-tagged proteins). In accordance with this protocol, an aliquot of previously prepared competent M15 cells was incubated with an aliquot of the ligation mixture in ice for 20 minutes and subsequently incubated at 42ºC for 90 minutes. Following said incubations, the cells were re-suspended in Psi broth and incubated for 90 minutes at 37ºC. Subsequently, the M15 cells were seeded in LB agar plates containing 25 micrograms per millilitre of kanamycin and 100 micrograms of ampicillin, and incubated overnight at 37ºC. A positive colony was allowed to grow in LB broth supplemented with kanamycin and penicillin, centrifuged, and the sedimented cells were stored in glycerol-containing SOB medium at -80ºC.

### EXAMPLE 4. Expression of the recombinant polypeptide of Anisakis simplex

In order to produce the recombinant polypeptide of recombinant *Anisakis* of Example 2, the transformed M15 cells were thawed and allowed to grow at 37ºC in LB broth, supplemented with kanamycin and ampicillin, with orbital stirring (200 rpm), until an OD₆₀₀ of 0.5 was reached. Subsequently, the expression of the polypeptide was induced by adding 1 mM IPTG, and the cells were incubated for 4 additional h at 37ºC under the same stirring conditions. Subsequently, the cells were centrifuged at 5000 x g and stored, frozen, at -30ºC, until purification of the expressed polypeptide was achieved, as described in Example 5. Under these culture conditions, the M15 cells produce the recombinant polypeptide of SEQ ID No.: 4 precipitated in the form of inclusion bodies.

### EXAMPLE 5. Purification of the recombinant polypeptide of Anisakis simplex

In a preferred embodiment, the inclusion bodies of the sedimented M15 cells, obtained from a 250-ml culture, were purified in accordance with the following steps: a) the bacterial sediment was re-suspended in 15 ml of B-PER reagent (Pierce, Rockford, IL, USA) and gently stirred for 10 minutes; b) the sample was centrifuged at 15.000 x g and the supernatant, which contained soluble proteins, was discarded; c) the sediment containing the inclusion bodies was re-suspended in another 15 ml of B-PER and lysozyme at a final concentration of 200 micrograms (Sigma-Aldrich, Madrid, Spain) was added to the cell suspension and incubated at ambient temperature for 5 minutes; d) the suspension was diluted with 100 ml of a 1:10 dilution of B-PER and the cells were re-suspended using a *vortex* agitator; e) the cell suspension was centrifuged at 15,000 x g for 15 minutes and the sediment was re-suspended in another 100 ml of B-PER and stirred in the *vortex;* f) the suspension containing diluted B-PER was centrifuged at 15,000 x g and the sediment was re-suspended in 50 ml of a denaturation buffer consisting of 100 mM NaH₂PO₄, 10 mM Tris.Cl and 6 M Urea, pH 8.0, and subsequently stirred in the *vortex*; g) the suspension was centrifuged at 15,000 x g for 20 minutes and dissolved in 10 ml of a denaturation buffer consisting of 100 mM NaH₂PO₄, 10 mM Tris.Cl and 8 M Urea, pH 8.0 (loading buffer); h) 2.5 ml of a 50% Ni-NTA resin suspension (Qiagen) were added to the solution containing the peptide, and the mixture was stirred in an orbital stirrer for 30 minutes at ambient temperature. Subsequently, the resin suspension was loaded in an empty column, and washed with two volumes of the loading buffer; i) the retained polypeptides were eluted with a solution containing 0.15 mM Tris.Cl, pH 10.5, which contained 250 mM imidazole, and stored, frozen, at -30ºC until they were to be used.

### EXAMPLE 6. Subcloning of the fragment of the Anisakis simplex cDNA sequence of SEQ ID No.: 17 in the pTARGET vector and transformation of competent JM109 cells.

In another preferred embodiment, an internal fragment of 834 amino acids (SEQ ID No.: 17) corresponding to the positions of nucleotides 1306-2139 of SEQ ID No.:1, which encodes the amino acid sequence of the polypeptide represented by SEQ ID No.: 18, was subcloned in the pTARGET vector (Promega). To do so, SEQ ID No.: 17 was amplified by PCR using a set of direct (5' SEQ ID No. 25 3') and reverse (5' SEQ ID No. 26 3') primers, and the transformed pQE-31 vector of EXAMPLE 3 as the template. The PCR conditions were: a denaturation cycle at 94ºC for 5 minutes, followed by 35 cycles of (45 s of denaturation at 94ºC; 45 seconds of hybridisation at 55ºC; and 1.5 minutes of elongation at 72ºC), and a final elongation cycle of 10 minutes at 72ºC. The PCR products were analysed in a 2% agarose gel and subsequently cloned in the pTARGET vector. The ligation of pTARGET and the *Anisakis* insert, and the transformation of competent JM109 cells were performed as specified in the product's instructions for use. Positive JM109 transformants were selected by white colony selection using LB agar plates supplied with ampicillin/IPTG/X-Gal, as described by the manufacturer.

Under the experimental conditions described, subcloning of the cDNA sequence corresponding to SEQ ID No.: 17 in the pTARGET vector led to a plasmid ORF of 846 nucleotides (SEQ ID No.: 5) that encodes the sequence of 282 amino acids of SEQ ID No.: 6, which includes the *Anisakis* polypeptide of SEQ ID No.: 17, and an additional sequence of 4 amino acids (SEQ ID No.: 24) located at the terminal carboxyl portion of SEQ ID No.: 17.

### EXAMPLE 7. Isolation of the recombinant plasmid DNA of JM109 cells and vaccination of mice with plasmid DNA

A positive colony of JM109 cells was allowed to grow in LB broth-ampicillin, centrifuged at 15,000 x g for 15 minutes, and the sediment used to extract the plasmid DNA using the Plasmid Maxi kit (Qiagen). A total of 50 microlitres (concentration of one microgram per microlitre) of endotoxin-free plasmid DNA were inoculated in the quadriceps muscle of the two legs of Balb/c mice 10-20 weeks of age. The inoculations were performed using an insulin syringe as described by Leiro et al. (2002).

### EXAMPLE 8. Immunoassays for the determination of anti-Anisakis antibodies in the sera of infected patients

An immunoassay in accordance with this invention may be any immunoassay capable of detecting the presence of anti-*Anisakis* antibodies in any biological fluid from an animal subject or species.

Examples of immunoassay types include liquid-phase and solid-phase immunoassays, and competitive and non-competitive immunoassays in a direct or indirect format.

Typically, the measurement of the levels of specific anti-*Anisakis* antibodies in a biological fluid, such as serum or plasma, may be performed using ELISA methods, such as the ELISA methods described in this example.

### a) Serum samples

The serum samples used in the ELISA methods of this example were obtained from non-infected subjects (healthy blood donours) and from subjects infected with the *Anisakis simplex* parasite. The sera were considered to be positive when they were obtained from subjects with a recent history of having eaten raw fish and the presence of the parasite was confirmed by endoscopic examination.

### b) Capture ELISA with O-deglycosylated antigens and UA3 monoclonal antibodies.

A capture ELISA method (UA3-ELISA) is described based on the capture of specific O-deglycosylated antigens of *Anisakis* spp. by immobilised UA3 monoclonal antibodies.

The *O*-deglycosylated antigen used in this immunoassay was obtained as described above, and is based on the treatment of intact antigens of Anisakis with 0.02 mM NaOH at 50ºC for 16 h, and additional neutralisation of the pH with HCl.

In order to perform the capture ELISA, the following steps were performed: a) coupling of purified UA3 monoclonal antibodies (100 microlitres/well; antibody concentration of 10 micrograms/ml) overnight; b) aspiration and, subsequently, washing of the ELISA plate with PBS three times; c) aspiration and, subsequently, blocking of the ELISA wells for 1 h at 37ºC with a PBS solution containing 3% powder skim milk and 0.2% Tween-20 (PBS-TM); d) aspiration and, subsequently, incubation for 1.5 h at 37ºC with 100 microlitres of non-diluted human serum; e) washing three times with 200 microlitres per well of PBS containing 0.2% Tween-20 (PBS-T); f) incubation with 100 microlitres of a 1:2,500 dilution of FITC-labelled human anti-IgE monoclonal antibodies (Ingenasa, Madrid, Spain; labelling with FITC was performed in accordance with the method described by Liddel and Cryer, 1991); g) aspiration and washing 3 times with 200 microlitres/well of PBS-T; h) aspiration and incubation with 100 microlitres of a 1:1,500 dilution of peroxidase-conjugated rabbit anti-FITC antibodies (Dako, Barcelona, Spain); i) aspiration and washing with 200 microlitres per well of PBS-T; j) aspiration and incubation with peroxidase substrate (Sigma Fast OPD, Sigma); k) reading of optical densities (OD) at 492 nm in a multi-well reader (Molecular Devices, Sunnyvale, CA).

### c) Indirect ELISA with the recombinant polypeptide of Anisakis expressed in transformed M15 cells.

In a preferred embodiment, the recombinant polypeptide of SEQ ID No.: 4 was used to perform an indirect ELISA in order to measure anti-*Anisakis* antibodies in the sera of subjects infected with *Anisakis.*

In this example, an ELISA method is described for the determination of IgE antibodies in human serum samples, but other classes (IgA, IgG, IgM) and subclasses (IgA1, IgA2, IgG1, IgG2, IgG3 and IgG4) of antibodies may also be measured by changing the specificity of the secondary anti-human antibodies. Similarly, the immunoassay may also be adapted for the determination of antibodies in biological fluids of other animal species (for example, other mammals, birds or fish), by selecting the appropriate secondary anti-species antibodies.

In a preferred embodiment, the method to perform the indirect ELISA (rUA3-ELISA) of this example was performed in accordance with the following steps: a) coupling of the ELISA plates with the recombinant antigen of *Anisakis* of SEQ ID No.: 4 in 0.1 M Tris.Cl, pH 10.5; b) aspiration and, subsequently, washing of the ELISA plate with PBS three times; c) aspiration and, subsequently, blocking of the ELISA wells for 1 h at 37ºC with a Tris-buffered saline solution (TBS) containing 3% powder skim milk and 0.2% Tween-20 (TBS-TM); d) aspiration and, subsequently, incubation of the plates for 1.5 h at 37ºC with 100 microlitres of non-diluted human serum; e) washing three times with 200 microlitres per well of TBS containing 0.2% Tween-20 (TBS-T); f) incubation with 100 microlitres of a 1:2,500 dilution of FITC-labelled human anti-IgE monoclonal antibodies (Ingenasa; labelled with FITC in accordance with the method described by Liddel and Cryer, 1991); g) aspiration and, subsequently, washing 3 times with 200 microlitres/well of TBS-T; h) aspiration and, subsequently, incubation with 100 microlitres of a 1:1,500 dilution of peroxidase-conjugated rabbit anti-FITC antibodies (Dako); i) aspiration and, subsequently, washing with 200 microlitres per well of PBS-T; j) aspiration and incubation with peroxidase substrate (Sigma Fast OPD, Sigma); k) reading of the optical densities (OD) at 492 nm in a multi-well reader (Molecular Devices).

The following Table I shows a comparison of the IgE values obtained for 22 positive serum samples from patients infected with the *Anisakis simplex* nematode, assayed by the capture ELISA (UA3-ELISA) and the indirect ELISA (rUA3-ELISA) methods described in Example 8 of this invention. The table shows the mean absorbance values, measured at 492 nm, for each serum. The cut-off values for the determinations of IgE were OD = 0.105 for the UA3-ELISA method and OD = 0.049 for the rUA3-ELISA method. Note that sample 2 was only positive for the rUA3-ELISA method. These results show that both the UA3-ELISA method (sensitivity of 95.45%) and the rUA3-ELISA method (sensitivity of 100%) are valid methods for the serodiagnosis of human infections by *Anisakis* spp.

**Table I**

| | **Optical Density (492 nm)** | |
|---|---|---|
| | **UA3-ELISA Method** | **rUA3-ELISA method** |
| **Sample 1** | 1.5 | 1.04 |
| **Sample 2** | 0.067 | 0.068* |
| **Sample 3** | 0.48 | 0.43 |
| **Sample 4** | 0.68 | 0.9 |
| **Sample 5** | 0.9 | 0.5 |
| **Sample 6** | 0.12 | 0.06 |
| **Sample 7** | 0.11 | 0.1 |
| **Sample 8** | 0.11 | 0.35 |
| **Sample 9** | 0.83 | 0.55 |
| **Sample 10** | 0.45 | 0.36 |
| **Sample 11** | 0.39 | 0.12 |
| **Sample 12** | 1.49 | 1.3 |
| **Sample 13** | 1.82 | 1.16 |
| **Sample 14** | 0.177 | 0.24 |
| **Sample 15** | 0.6 | 0.35 |
| **Sample 16** | 0.17 | 0.06 |
| **Sample 17** | 0.21 | 0.149 |
| **Sample 18** | 0.84 | 0.55 |
| **Sample 19** | 0.22 | 0.11 |
| **Sample 20** | 0.28 | 0.79 |
| **Sample 21** | 0.6 | 0.28 |
| **Sample 22** | 0.88 | 0.83 |

### EXAMPLE 9. Production of antibodies in mice transfected with the recombinant pTARGET vector that encodes the polypeptide of SEQ ID No.: 6

In this example, blood was extracted, under anaesthesia, from mice transfected with recombinant pTARGET that encoded the polypeptide of SEQ ID No.: 6 (see EXAMPLE 7), and the serum obtained was assayed in indirect ELISA in order to determine the presence of reactive IgG1 antibodies with the polypeptide of SEQ ID No.: 4 immobilised on the ELISA plate. The ELISA method was the same as that described in EXAMPLE 8b, except that peroxidase-labelled goat mouse anti-IgG antibodies (Caltag Laboratories, Burlingame, CA, USA; 1:3,000 dilution) were used instead of secondary anti-human reagents. In this example, mouse sera at a 1:100 dilution were assayed.

The following Table II shows the response of IgG1 antibodies obtained in BALB/c mice following vaccination (by parenteral route) with 100 micrograms/mouse of the recombinant *p*-TARGET plasmid containing the DNA insert of *Anisakis simplex* that encodes the polypeptide of SEQ ID No.: 6. The extraction of blood from the mice was performed one month after the immunisation. The table shows the mean absorbance values ± SD for each serum, measured at 492 nm (three independent determinations).

These results show that a recombinant DNA vaccine containing a DNA sequence of *Anisakis* (shown in this experiment by SEQ ID No.: 6 of this invention) may be useful for the *in vivo* induction of anti-*Anisakis* antibodies. The *in vivo* induction of antibodies by vaccines is of interest for:
a) the induction of immunological protection against infectious agents, and
b) the prevention of allergic reactions.

**Table II**

| | **Absorbance at 492 nm** |
|---|---|
| Mouse 1 (*p*TARGET without *Anisakis* insert) | 0.0 ± 0.01 |
| Mouse 2 (*p*TARGET plus *Anisakis* insert of SEQ ID No.: 5) | 0.27 ± 0.05 |
| Mouse 3 (*p*TARGET plus *Anisakis* insert of SEQ ID No.: 5) | 0.45 ± 0.07 |

### EXAMPLE 10. Labelling and applications of recombinant peptides and polypeptides of Anisakis simplex of this invention

Labelling of the peptides and polypeptides of this invention with a detectable biomolecule or chemical molecule is useful for purposes such as *in vivo* and *in vitro* diagnosis and laboratory research. There are many different labels and methods known by those skilled in the art (for example, Kessler (Ed). Nonradioactive labeling and detection of biomolecules. Springer-Verlag, Berlin 1992); Walker (Ed). The protein protocols handbook. Humana Press, Totowa, New Jersey (1996)).

Examples of the types of labels that may be used in this invention include enzymes, radioisotopes, fluorescent compounds, chemiluminescent compounds, bioluminescent compounds and chromogenic substances, including coloured particles such as colloidal gold and latex particles.

In a preferred embodiment, the peptides and polypeptides of this invention are radiolabelled with, but not limited thereto, ³²P, ¹⁴C, ³H, ³⁵S, ¹²⁵I or ¹³¹I. The labelled peptides may be detected by methods such as scintillation count, gamma-ray spectrometry or autoradiography.

In another preferred embodiment, the peptides and polypeptides of this invention may be labelled with firefly luciferin. The bioluminescent compound may be covalently bound to the selected peptide or polypeptide by conventional methods, and the labelled peptide or polypeptide may be detected when an enzyme, for example, luciferase, catalyses a reaction with ATP that causes the bioluminescent molecule to emit light photons.

Fluorogens are also broadly used as labels. Examples of fluorogens include fluorescein and derivatives, rhodamine, Texas Red, phycoerithrin, phycocyanin and others. In general, the fluorogen molecules are detected by means of a fluorescence detector.

In another preferred embodiment, the peptides and polypeptides of this invention (for example, the polypeptide of SEQ ID No.: 4) may be labelled with biotin and captured in a liquid support by a specific biotin ligand such as avidin or streptavidin. Once the labelled polypeptide has been immobilised on the solid support (for example, the well of an ELISA plate), it may be used to detect antibodies against the selected polypeptide by performing the typical incubations and washing steps, such as those described in Example 8b of this invention.

As an alternative, the peptides and polypeptides of this invention may be labelled with enzymes (for example, peroxidase, alkaline phosphatase and other enzymes that provide a chromogenic or fluorogenic reaction after the addition of the appropriate substrate). Peptides and polypeptides labelled with selected enzymes may be used, for example, to detect specific antibodies that recognise said peptides or polypeptides in a capture ELISA method, where the antibody to be detected is first captured by another antibody immobilised on a solid support and the labelled peptide or polypeptide, plus the appropriate substrate, are used to show that the antibody-polypeptide bond has been formed.

In another preferred embodiment, the peptides and polypeptides of this invention may be labelled with colloidal gold to be used in lateral flow immunochromatography assays, in accordance with methods that are well known to those skilled in the art. For example, methods of producing gold particles of various sizes and for the conjugation of gold with proteins are described in: Dykstra (Ed). A manual of applied techniques for biological electron microscopy. Plenum Press. New York (1993).

Moreover, in another preferred embodiment, the labelling molecule may be magnetic microparticles and the immobilised system may be a magnet.

### EXAMPLE 11. Labelling and applications of Anisakis simplex nucleic acid molecules of this invention.

Labelled nucleic acid molecules may be used as probes to detect complementary target sequences in a complex nucleic acid mixture by specific hybridisation methods such as Southern transfer, Northern transfer, slot blot and dot blot, *in situ* hybridisation (ISH) and microarrays. The methods of labelling nucleic acid molecules are well known in the state of the art and may be performed using different labelling agents, including, but not limited thereto: radioactive agents, fluorescent colouring agents, chemiluminescent agents; microparticles; enzymes; colorimetric labels (such as, for example, colouring agents, colloidal gold and similar); magnetic labels and haptens (such as, for example, biotin, dioxigenin (DIG) and others for which antisera or monoclonal antibodies are available).

The labelled probes of this invention may be used as diagnostic tools, for example, for the detection of *Anisakis* spp. DNA in human and/or animal infected tissues, and for research.

In a preferred embodiment, the selected nucleic acid sequences of this invention may be labelled with DIG by PCR amplification using DIG-labelled nucleotides (Boehringer-Mannheim, Germany).

As an example, a selected DIG-labelled nucleotide sequence probe (482 base pairs), corresponding to the positions of nucleotides 1490 and 1971 of SEQ ID No.: 1, may be obtained using the transformed pQE-31 vector of EXAMPLE 3 as a template, and a set of direct (5' SEQ ID No.: 27 3') and reverse (5' SEQ ID No.: 28 3') primers. The tissue samples to be analysed, obtained from the biopsies of infected humans or animals, may be fixated in 4% paraformaldehyde, and subsequently analysed to detect the presence of *Anisakis* spp. DNA using ISH methods and the above-mentioned DIG-labelled probe. A detailed description of a method of performing the ISH of this example has been provided by Leiro et al. (2001), which was incorporated into this document as a reference.

### EXAMPLE 12. Analysis of synthetic peptide sequences that inhibit the binding of monoclonal antibody US3 to Anisakis simplex peptides and polypeptides of this invention.

In one aspect of this invention, peptide sequences are described that are recognised by monoclonal antibody UA3. Said peptide sequences were recognised as "repetitions" of amino acids by analysing the amino acid sequence of SEQ ID No.: 2 using the RADAR programme, http://www.ebi.ac.uk/Radar/, at the EMBL-EBI (European Bioinformatics Institute). Selected sequences of 12 amino acids were chemically synthesised from these "repetitions" and assayed in competitive ELISA regarding the inhibition of the binding of UA3 monoclonal antibodies to the recombinant polypeptide of SEQ ID No.: 4, immobilised in the wells of an ELISA plate.

*The inhibitory activity of peptides corresponding to amino acid sequences No.: 7-11 was assayed in a capture ELISA, in accordance with the following typical steps: a) binding of the polypeptide of SEQ ID No.: 4 to the wells of an ELISA plate overnight; b) aspiration and, subsequently, blocking of the ELISA wells for 1 h at 37ºC with TBS buffer containing 3% powder skim milk and 0.2% Tween-20 (TBS-TM); d) aspiration and, subsequently, incubation for 2 h at 37ºC with 100 microlitres of the appropriate dilution of MAb UA3 pre-incubated with the inhibitory peptide to be assayed; d) washing 3 times with 200 microlitres*/*well of TBS containing 0.2% Tween-20 (TBS-T); e) incubation with 100 microlitres of a 1:3,000 dilution of peroxidase-conjugated goat anti-mouse IgG (Nordic Immunological Laboratory, Tilburg, The Netherlands); f) aspiration and washing 3 times with 200 microlitres*/*well of TBS-T; g) aspiration and washing with 200 microlitres*/*well of PBS-T; h) aspiration and incubation with peroxidase substrate (Sigma Fast OPD, Sigma); i) reading of the optical densities (OD) at 492 nm in a multi-well reader (Molecular Devices).*

The following Table III shows the inhibition values obtained for the synthetic peptides of SEQ ID No.: 7-11 (assayed at three concentrations) on the binding of monoclonal antibody UA3 to the polypeptide of SEQ ID No.: 4 immobilised on an ELISA plate. The results were expressed as the percentage of inhibition for each peptide dilution with respect to the control (no inhibition). ND: Not performed. As deduced from the results observed, monoclonal antibody UA3 has a preference for peptides of SEQ ID No.: 7 and 8, which differ by only one amino acid.

**Table III**

| | **Peptide concentration** | | |
|---|---|---|---|
| | **2x10⁻³ M** | **7.4x10⁻⁵ M** | **8.23x10⁻⁶ M** |
| **(SEQ ID No. 7)** | 98.45 | 89.79 | 73.25 |
| **(SEQ ID No. 8)** | 97.79 | 79.75 | 63.63 |
| **(SEQ ID No. 9)** | 76.75 | 17.96 | 3.42 |
| **(SEQ ID No. 10)** | ND | 11.42 | 0 |
| **(SEQ ID No. 11)** | 26.04 | 0 | 0 |

### EXAMPLE 13. Significance of the synthetic amino acid sequences of SEQ ID No.: 7-11 as a target for anti-Anisakis IgE antibodies present in the sera of infected patients

In another preferred embodiment, the inhibitory activity of the amino acid sequences (SEQ ID No.: 7-11) on the binding of human anti-*Anisakis* IgE antibodies to the polypeptide of SEQ ID No.: 4 was assayed in an indirect competitive ELISA, in the following manner: a) binding of the polypeptide of SEQ ID No.: 4 to the wells of an ELISA plate overnight; b) aspiration and, subsequently, blocking of the ELISA wells for 1 h at 37ºC with TBS buffer containing 3% powder skim milk and 0.2% Tween-20 (TBS-TM); d) aspiration and, subsequently, incubation for 2 h at 37ºC with 100 microlitres of a 1:1 dilution of the corresponding patient's serum and the peptide solution in TBS (1.5 X 10⁻³ M, final concentration for each of the above-mentioned peptides); d) washing 3 times with 200 microlitres/well of TBS containing 0.2% Tween-20 (TBS-T); e) incubation with 100 microlitres of a 1:2,500 dilution of FITC-labelled human anti-IgE monoclonal antibodies; f) aspiration and, subsequently, washing 3 times with 200 microlitres/well of TBS-T; g) aspiration and, subsequently, incubation with 100 microlitres of a 1:1,500 dilution of peroxidase-conjugated rabbit anti-FITC antibodies (Dako); h) aspiration and, subsequently, washing with 200 microlitres/well of PBS-T; i) aspiration and incubation with peroxidase substrate (Sigma Fast OPD, Sigma); j) reading of the optical densities (OD) at 492 nm in a multi-well reader (Molecular Devices).

The following Table IV shows the inhibitory activity of a mixture of the synthetic peptides corresponding to SEQ ID No.: 7-11 on the binding of IgE antibodies present in the sera of five infected patients to the polypeptide of SEQ ID No. 4 immobilised on ELISA plates. The peptide mixture was assayed at a final concentration of 1.5 x 10⁻³ M for each peptide. The results were expressed as percentages of inhibition of the peptide mixture with respect to the control (inhibited with a mixture of irrelevant peptides at the same final concentration). As observed in the Table, the assayed sequences were targets for a range of 25-74 percent of all the anti*-Anisakis* IgE antibodies present in the sera of infected patients. However, it is understood that probably other recombinant or synthetic peptides and polypeptides that comprise a stretch of 8 or more adjacent amino acids of SEQ ID No.: 2, or repetitions of said sequences, may also be recognised by anti*-Anisakis* antibodies of several isotypes that are present in the sera of infected patients.

**Table IV**

| | **Percentage of inhibition** |
|---|---|
| **Patient 1** | 53.5 |
| **Patient 2** | 74.1 |
| **Patient 3** | 35.0 |
| **Patient 4** | 30.4 |
| **Patient 5** | 25.4 |

### EXAMPLE 14. Characterisation of antigens of Anisakis larvae in biological tissues

In a preferred embodiment, the peptides or polypeptides of this invention may be used to immunise a mammal (such as, for example, rabbits, sheep, goats, mice or rats) and the specific polyclonal antibodies obtained may be used to show the presence of *Anisakis* larvae in infected tissues of human beings or animals. Typically, the procedure to reveal antigens of *Anisakis* in tissues with parasites may be performed following a conventional immunohistochemical method in accordance with the following general steps: a) preparation of the slides containing the tissue sections included in paraffin; b) blocking of the tissues with TBS-TM or any other blocking reagent; c) washing step; d) incubation with anti*-Anisakis* polyclonal antibodies; e) washing step; f) incubation with labelled secondary antibodies that recognise IgG antibodies of the animal species used for the immunisation (in this example, the preferred labelling molecule is the peroxidase enzyme, but any other label may be used to label secondary antibodies, such as, for example, those specified in EXAMPLE 10 of this invention); g) washing step; h) incubation with the appropriate enzyme substrate (such as, for example H₂O₂ ± DAB); i) microscope observation.

In another preferred embodiment, the presence of *Anisakis* spp. proteins that comprise the amino acid sequences of this invention in infected tissues of human beings or animals may be revealed using monoclonal antibody UA3 as the primary antibody, by conventional immunohistochemistry methods.

### BIBLIOGRAPHY

Akao, N., Yoshimura, H. (1989). Latex agglutination test for immunodiagnosis of gastric anisakiasis. In: Gastric anisakiasis in Japan. Epidemiology, diagnosis, treatment. (Eds. Ishikura, H. and Namiki, M.), Springer-Verlag, Tokyo, pp.: 97-102.
Asaishi, K.; Nishino, C., Hayasaka, H. (1989). Passive hemagglutination test (Boyden). In: Intestinal anisakiasis in Japan. Infected fish, sero-immunological diagnosis, and prevention. (Eds. Ishikura, H. and Kikuchi, K.) Springer-Verlag, Tokyo, pp.: 167-172.
Ausubel, F.M., Brent, R., Kingston, R.E., Moore, D.D., Seidman, J.G., Smith, J.A., Struhl, K. (Eds) (1998). Current protocols in molecular biology. John Willey & Sons Inc. New York.
Baeza, M.L., Rodriguez, A., Matéu, V., Rubio, M., Tornero, P., de Barrio, M., Herrero, T., Santaolalla, M., Zubeldia, J.M. (2004). Characterization of allergens secreted by Anisakis simplex parasite: clinical relevance in comparison with somatic allergens. Clinical and Experimental Allergy 34: 296-302.
Campana-Rouget, Y., Biocca, E. (1955). A new species of Anisakis in a Mediterranean seal. Annales de Parasitologie Humaine et Comparée 30: 477-480.
Daschner, A., Alonso-Gómez, A., Cabanas, R., Suárez-de-Parga, J.M., López-Serrano, M.C. (2000). Gastroallergic anisakiasis: borderline between food allergy and parasitic disease-clinical and allergologic evaluation of 20 patients with confirmed acute parasitism by Anisakis simplex. Journal of Allergy and Clinical Immunology 105: 176-81.
Del Pozo, M.D., Moneo, I., Fernández de Corres, L., Audícana, M.T., Muñoz, D., Fernández, E., Navarro, J.A., García, M. (1996). Laboratory determinations in Anisakis simplex allergy. Journal of Allergy and Clinical Immunology, 97: 977-984.
Desowitz, R.S.; Raybourne, R.B., Ishikura, H., Kliks, M.M. (1985). The radioallergosorbent test (RAST) for the serological diagnosis of human anisakiasis. Transactions of the Royal Society of Tropical Medicine and Hygiene, 79: 256-259.
Deshpande S.S. (1996) Enzyme immunoassays, from concept to product development, Chapman & Hall, New York.
Dykstra, M.J. (Ed). (1993). A manual of applied techniques for biological electron microscopy. Plenum Press. New York.
Dollfus, R. (1966). Helminthofauna de Kogia breviceps (de Blainville, 1838) cetace odeontocete. Annales de la Societé des Sciences Naturelles de la Carente-Maritime 4: 3-6.
García, M., Moneo, I., Audícana, M.T., Del Pozo, M.D., Muñoz, D., Fernández, E., Díez, J., Etxenagusia, M.A., Ansotegui, J., Fernández de Corres, L. (1997). The use of IgE immunoblotting as a diagnostic tool in Anisakis simplex allergy. Journal of Allergy and Clinical Immunology, 99: 497-501.
Gasteiger E., Gattiker A., Hoogland C., Ivanyi I., Appel R.D., Bairoch, A. (2003). ExPASy: the proteomics server for in-depth protein knowledge and analysis. Nucleic Acids Res. 31:3784-3788.
Huang, X., Miller, M. (1991). A time-efficient, linear-space local similarity algorithm. Advances in Applied Mathematics 12: 337-357 (1991).
Kessler, C. (1992) Nonradioactive labeling and detection of biomolecules. Springer-Verlag, Berlin 1992).
Leiro, J., Iglesias, R., Ubeira, F.M., Sanmartin, M.L. (2001). Non-isotopic detection of Tetramicra brevifilum (Microspora) DNA in turbot tissues. Journal of Parasitology, 87: 1488-90.
Leiro, J., Siso, M.I., Iglesias, R., Ubeira, F.M., Sanmartin, M.L. (2002). Mouse antibody response to a microsporidian parasite following inoculation with a gene coding for parasite ribosomal RNA. Vaccine, 20: 2648-55. Liddell, J.E., Cryer, A. (1991). Characterization, purification and labelling. In: A practical guide to monoclonal antibodies. Wiley & Sons, Chichester, pp.: 105-138.
Mattiucci, S., Paggi, L., Nascetti, G., Santos, C.P., Costa, G., Di Beneditto, A.P., Ramos, R., Argyrou, M., Cianchi, R., Bullini, L. (2002). Genetic markers in the study of Anisakis typica (Diesing, 1960): larval identification and genetic relationship with other species of Anisakis Dujardin, 1845 (Nematoda: Anisakidae). Systematic Parasitology 51: 159-170.
Mattiucci, S., Nascetti, G., Dailey, M., Webb, S.C., Barros, N.B., Cianchi, R., Bullini, L. (2005). Evidence for a new species of Anisakis (Dujardin, 1845): morphological description and genetic relationship between congeners (Nematoda: Anisakidae). Systematic Parasitology 61: 157-171.
Nascetti, G., Paggi, L., Orecchia, P., Smith, J.W., Mattiucci, S., Bullini, L. (1998). Electrophoretic studies on the Anisakis simplex complex (Ascaridida: Anisakidae) from the Mediterranean and North East Atlantic. International Journal for Parasitology 16: 633-640.
Oshima, T. (1972). Anisakis and anisakiasis in Japan and adjacent area. In: Progress of Medical Parasitology in Japan, Vol. IV. (Eds. Morishita, K.; Kojima, Y. and Matsubayashi, H.), Meguro Parasitological Museum, Tokyo, pp.: 301-393.
Paggi, L., Nascetti, G., Webb, S.C., Mattiucci, S., Cianchi, R., Bullini, L. (1998). A new species of Anisakis Dujardin, 1845 (Nematoda, Anisakidae) from beaked whales (Ziphiidae): allozyme and morphological evidence. Systematic Parasitology 40: 161-174.
Petithory, J.C.; Lapierre, J., Rousseau, M., Clique, M.T. (1986). Diagnostic sérologique de l'anisakiase (granulome éosinophile digestif) par précipitation en milieu gélifié (Ouchterlony, électrosynérèse, immunoélectrophorèse). Médecine et Maladies Infectieuses, 3: 157-162.
Poggensee, U.; Schomme, G.; Jansen-Rosseck, R., Feldemeier, H. (1989). Immunodiagnosis of human anisakiasis by use of larval excretory-secretory antigen. Zentralblatt für Bakteriologie und Microbiologie und Hygiene, A270: 503-510.
Rodero, M., Jiménez, A., Cuéllar, C. (2002). Evaluation by ELISA of Anisakis simplex larval antigen purified by affinity chromatography. Mem Inst Oswaldo Cruz. 97: 247-52.
Price, C.P., Newman, D.J. (Eds) (1997). Principles and Practice of Immunoassay, Macmillan Reference LTD, New York.
Sambrook, J., Russel, D.W. (Eds). (2001). Molecular cloning, a laboratory manual. Cold Spring Harbor Laboratory Press, New York.
Tatusova, T.A., Madden, T.L. (1999). Blast 2 sequences - a new tool for comparing protein and nucleotide sequences. FEMS Microbiological Letters. 174: 247-250.
Tijssen, P. (1985) Practice and theory of enzyme immunoassays. In: Laboratory Techiniques in Biochemistry and Molecular Biology (R.H. Burdon and PH van Knippenberg, eds), Elsevier, Amsterdam.
Tsuji, M. (1989). Serological and immunological studies. In: Gastric anisakiasis in Japan. Epidemiology, diagnosis, treatment. (Eds. Ishikura, H. and Namiki, M.), Springer-Verlag, Tokyo, pp.: 89-95.
Tsuji, M. (1990). Ouchterlony test and immunoelectrophoresis. In: Intestinal anisakiasis in Japan. Infected fish, sero-immunological diagnosis, and prevention. (Eds. Ishikura, H. and Kikuchi, K.) Springer-Verlag, Tokyo, pp.: 183-186.
Ubeira, F.M., Iglesias, R. (2000). Monoclonal antibodies in the study of Anisakis simplex. Allergy 55 (Suppl 59): 18-28.
Van Thiel, P.H. (1960). A nematode parasitic to herring causing acute abdomen syndromes in man. Tropical and Geographical Medicine 2: 97-113.
Yagihashi, A., Sato, N., Takahashi, S., Ishikura, H., Kikuchi, K. (1990). A serodiagnostic assay by microenzyme-linked immunosorbent assay for human anisakiasis using a monoclonal antibody specific for Anisakis larvae antigen. The Journal of Infectious Diseases, 161: 995-998.
Yamamoto, Y.; Nakata, H., Yamamoto, Y. (1990). Detection of anti-Anisakis antibody of IgE type in sera of patients with intestinal anisakiasis. In: Intestinal anisakiasis in Japan. Infected fish, sero-immunological diagnosis, and prevention. (Eds. Ishikura, H. and Kikuchi, K.) Springer-Verlag, Tokyo, pp.: 205-216.

## Claims

1. An isolated nucleic acid sequence selected from the following group:
a. A nucleic acid that comprises SEQ ID No. 1.
b. A nucleic acid that comprises the complement of SEQ ID No. 1.
c. A fragment of the nucleic acid of SEQ ID No. 1 that comprises SEQ ID No. 15 or SEQ ID No. 17.

2. An isolated nucleic acid sequence selected from the following group:
- (i) The nucleic acid comprises a fragment of SEQ ID No. 1 that encodes a stretch of 10 or more adjacent amino acids of SEQ ID No. 2 capable of being recognised by *anti-Anisakis* antibodies, or
- (ii) The nucleic acid comprises a fragment of SEQ ID No. 1 that encodes a stretch of 12 or more adjacent amino acids of SEQ ID No. 2 capable of being recognised by *anti-Anisakis* antibodies.

3. An isolated polypeptide, peptide or protein encoded by any of the isolated nucleic acid sequences of claim 1, selected from the following group:
a. A polypeptide, protein or peptide that comprises SEQ ID No. 2.
b. A fragment of SEQ ID No. 2 that comprises SEQ ID No. 16 or SEQ ID No. 18.

4. The isolated polypeptide, peptide or protein encoded by the nucleic acid of claim 2, wherein
- (i) The fragment of SEQ ID No. 2 comprises a stretch of 10 or more adjacent amino acids capable of being recognised by anti-*Anisakis* antibodies, or
- (ii) The fragment of SEQ ID No. 2 comprises a stretch of 12 or more adjacent amino acids capable of being recognised by anti-*Anisakis* antibodies.

5. The isolated peptide, polypeptide or protein in accordance with the claim 3(b) having an identity of at least 70% with the fragment of SEQ ID No. 2 that comprises SEQ ID No. 16 or SEQ ID No. 18; or in accordance with the claim 4(ii), having an identity of at least 90% with this fragment.

6. The isolated peptide, polypeptide or protein in accordance with the claim 3(b), which has an identity of at least 80% or at least 90% with the fragment of SEQ ID No. 2 that comprises SEQ ID No. 16 or SEQ ID No. 18.

7. An isolated peptide, polypeptide or protein, or any nucleic acid sequence encoding the amino acidic sequence thereof, that comprises an amino acid sequence, selected from the following group:
a. An amino acid sequence that comprises SEQ ID No. 7.
b. An amino acid sequence that comprises SEQ ID No. 8.
c. An amino acid sequence that comprises SEQ ID No. 9.
d. An amino acid sequence that comprises SEQ ID No. 10.
e. An amino acid sequence that comprises SEQ ID No. 11.
f. An amino acid sequence that comprises SEQ ID No. 12.
g. An amino acid sequence that comprises SEQ ID No. 13.
h. An amino acid sequence that comprises SEQ ID No. 14.

8. A peptide, polypeptide or protein, or any nucleic acid sequence encoding the amino acidic sequences thereof, that comprises two or more of the amino acid sequences of any of claims 3-7.

9. An isolated nucleic acid sequence or amino acid sequence in accordance with any of the preceding claims, where said nucleic acid or amino acid sequence is labelled with a signalling molecule.

10. The isolated nucleic acid sequence or amino acid sequence in accordance with claim 9, where said labelling molecule is selected from the following group:
a. Radioactive
b. Enzymatic
c. Fluorescent
d. Luminescent
e. Chemiluminescent
f. Haptens
g. Coloured

11. The isolated nucleic acid sequence or amino acid sequence in accordance with the preceding claim, where said labelling molecule is selected from the following group:
a. Biotin or the derivatives thereof
b. Nitrophenol derivative
c. Colloidal gold and
d. Latex

12. An isolated peptide, polypeptide or protein in accordance with any of claims 3-8, chemically fused or bound to an additional peptide, polypeptide or protein.

13. An expression vector or expression system that comprises a nucleotide sequence in accordance with any of claims 1, 2, 5-8.

14. An isolated prokaryotic or eukaryotic host cell transformed or transfected with a nucleic acid sequence of any of claims 1, 2, 5-11, or with the vector or expression system of claim 13.

15. A method of preparing a peptide, polypeptide or protein in accordance with any of claims 3-8 or 12, by any of the following techniques:
a. Recombinant techniques
b. Chemical synthesis
c. Substantial purification of nematodes of the family Anisakidae

16. An *in vitro* method of detecting antibodies against *Anisakis* spp. in a biological sample that comprises:
a. placing the biological sample, preferably serum, in contact with a peptide, polypeptide or protein of any of claims 3-8, under sufficient conditions to form an immunological complex between said polypeptide and the antibodies in the sample, and
b. detecting said immunological complex.

17. The method of claim 16, wherein said method is an immunoassay or an enzymatic immunoassay.

18. An immunological composition that comprises a peptide, polypeptide or protein in accordance with any of claims 3-8 or 12, or a nucleotide sequence in accordance with any of claims 1, 2, 5-8.

19. Use of a peptide, polypeptide or protein in accordance with any of claims 3-8 or 12, in the manufacturing of a drug for the treatment of allergy to *Anisakis* spp.

20. A suitable kit for the detection of anti-*Anisakis* spp. antibodies in a biological sample that comprises a peptide, polypeptide or protein in accordance with any of claims 3-8 or 12.

## Patentansprüche

1. Eine isolierte Nukleinsäuresequenz, ausgewählt aus der folgenden Gruppe:
a. Eine Nukleinsäure, welche die SEQ ID NO 1 umfasst.
b. Eine Nukleinsäure, welche das Komplement der SEQ ID NO 1 umfasst.
c. Ein Fragment der Nukleinsäure der SEQ ID NO 1, die die SEQ ID NO 15 oder SEQ ID NO 17 umfasst.

2. Eine isolierte Nukleinsäuresequenz, ausgewählt aus der folgenden Gruppe:
- (i) Die Nukleinsäure umfasst ein Fragment der SEQ ID NO 1, die einen Strang von 10 oder mehr angrenzenden Aminosäuren der SEQ ID NO 2 kodiert, welche von anti-Anisakiasis-Antikörpern erkannt werden können, oder
- (ii) Die Nukleinsäure umfasst ein Fragment der SEQ ID NO 1, die einen Strang von 12 oder mehr angrenzenden Aminosäuren der SEQ ID NO 2 kodiert, welche von anti-Anisakiasis-Antikörpern erkannt werden können.

3. Ein isoliertes Polypeptid, Peptid oder Protein, das von einer der isolierten Nukleinsäuresequenzen nach Anspruch 1 kodiert ist, ausgewählt aus der folgenden Gruppe:
a. Ein Polypeptid, Protein oder Peptid, welches die SEQ ID NO 2 umfasst.
b. Ein Fragment der SEQ ID NO 2, die die SEQ ID NO 16 oder SEQ ID NO 18 umfasst.

4. Das isolierte Polypeptid, Peptid oder Protein, das von der Nukleinsäure nach Anspruch 2 kodiert ist, wobei
- (i) Das Fragment der SEQ ID NO 2 einen Strang von 10 oder mehr angrenzenden Aminosäuren umfasst, welche von anti-Anisakiasis-Antikörpern erkannt werden können, oder
- (ii) Das Fragment der SEQ ID NO 2 einen Strang von 12 oder mehr angrenzenden Aminosäuren umfasst, welche von anti-Anisakiasis-Antikörpern erkannt werden können.

5. Das isolierte Peptid, Polypeptid oder Protein nach Anspruch 3 (b), welche zu mindestens 70 % mit dem Fragment der SEQ ID NO 2 übereinstimmt, die die SEQ ID NO 16 oder SEQ ID NO 18 umfasst; oder nach Anspruch 4 (ii), welche zu mindestens 90 % mit diesem Fragment übereinstimmt.

6. Das isolierte Peptid, Polypeptid oder Protein nach Anspruch 3 (b), welche zu mindestens 80 % oder zu mindestens 90 % mit dem Fragment der SEQ ID NO 2 übereinstimmt, die die SEQ ID NO 16 oder SEQ ID NO 18 umfasst.

7. Ein isoliertes Peptid, Polypeptid oder Protein, oder irgendeine andere Nukleinsäuresequenz, welch die Aminosäuresequenz davon kodiert, welche eine Aminosäuresequenz umfasst, die aus der folgenden Gruppe ausgewählt ist:
a. Eine Aminosäuresequenz, welche die SEQ ID NO 7 umfasst.
b. Eine Aminosäuresequenz, welche die SEQ ID NO 8 umfasst.
c. Eine Aminosäuresequenz, welche die SEQ ID NO 9 umfasst.
d. Eine Aminosäuresequenz, welche die SEQ ID NO 10 umfasst.
e. Eine Aminosäuresequenz, welche die SEQ ID NO 11 umfasst.
f. Eine Aminosäuresequenz, welche die SEQ ID NO 12 umfasst.
g. Eine Aminosäuresequenz, welche die SEQ ID NO 13 umfasst.
h. Eine Aminosäuresequenz, welche die SEQ ID NO 14 umfasst.

8. Ein Peptid, Polypeptid oder Protein oder eine beliebige andere Nukleinsäuresequenz, die die Aminosäuresequenz davon kodiert, welche zwei oder mehr Aminosäuresequenzen nach einem der Ansprüche 3 bis 7 umfasst.

9. Eine isolierte Nukleinsäuresequenz oder Aminosäuresequenz nach einem der vorhergehenden Ansprüche, wobei die besagte Nukleinsäure- oder Aminosäuresequenz mit einem Signalmolekül **gekennzeichnet** ist.

10. Die isolierte Nukleinsäuresequenz oder Aminosäuresequenz nach Anspruch 9, wobei besagtes Signalmolekül aus der folgenden Gruppe ausgewählt ist:
a. Radioaktiv
b. Enzymatisch
c. Fluoreszent
d. Lumineszierend
e. Chemilumineszierend
f. Hapten
g. Gefärbt

11. Die isolierte Nukleinsäuresequenz oder Aminosäuresequenz nach einem der vorhergehenden Ansprüche, wobei besagtes Signalmolekül aus der folgenden Gruppe ausgewählt ist:
a. Biotin oder Derivate davon
b. Nitrophenolderivat
c. Kolloidales Gold und
d. Latex

12. Ein isoliertes Peptid, Polypeptid oder Protein nach einem der Ansprüche 3 bis 8, das mit einem weiteren Peptid, Polypeptid oder Protein chemisch verschmolzen oder verbunden ist.

13. Ein Expressionsvektor oder ein Expressionssystem, das eine Nukleotidsequenz nach einem der Ansprüche 1, 2, 5 bis 8 umfasst.

14. Eine prokaryotische oder eukaryotische Wirtszelle, welche transformiert oder transfiziert ist, mit einer Nukleinsäuresequenz nach einem der Ansprüche 1, 2, 5 bis 11, oder mit dem Vektor oder dem Expressionssystem nach Anspruch 13.

15. Ein Verfahren zur Zubereitung eines Peptids, Polypeptids oder Proteins nach einem der Ansprüche 3 bis 8 oder 12, durch eine der folgenden Techniken:
a. Rekombinante Techniken
b. Chemische Synthese
c. Substanzielle Reinigung von Fadenwürmern der Familie der Anisakidae.

16. Ein *In-vitro-Verfahren* zur Feststellung von Antikörpern gegen *Anisakiasis* spp. in einer biologischen Probe, welches Folgendes umfasst:
a. Platzieren einer biologischen Probe, vorzugsweise ein Serum, in Kontakt mit einem Peptid, Polypeptid oder Protein nach einem der Ansprüche 3 bis 8, unter ausreichenden Bedingungen, um einen immunologischen Komplex zwischen dem besagten Polypeptid und den Antikörpern in der Probe zu bilden, und
b. Feststellen des besagten immunologischen Komplexes.

17. Das Verfahren nach Anspruch 16, wobei die besagte Methode ein Immunoassay oder ein Enzymimmunoassay ist.

18. Eine immunologische Zusammensetzung, die ein Peptid, Polypeptid oder Protein nach einem der Ansprüche 3 bis 8 oder 12, oder eine Nukleotidsequenz nach einem der Ansprüche 1, 2, 5 bis 8 umfasst.

19. Verwendung eines Peptids, Polypeptids oder Proteins nach einem der Ansprüche 3 bis 8 oder 12 bei der Herstellung eines Arzneimittels zur Behandlung von Allergien gegen *Anisakiasis* spp.

20. Ein geeignetes Kit zur Feststellung von *anti-Anisakiasis* spp. Antikörpern in einer biologischen Probe, das ein Peptid, Polypeptid oder Protein nach einem der Ansprüche 3 bis 8 oder 12 umfasst.

## Revendications

1. Une séquence d'acide nucléique isolée sélectionnée dans le groupe suivant :
a. Un acide nucléique comprenant la SEQ ID n° 1.
b. Un acide nucléique comprenant le complément de la SEQ ID n° 1.
c. Un fragment de l'acide nucléique de la SEQ ID n° 1 comprenant la SEQ ID n° 15 ou la SEQ ID n° 17.

2. Une séquence d'acide nucléique isolée sélectionnée dans le groupe suivant :
- (i) L'acide nucléique comprend un fragment de la SEQ ID n° 1 codant pour un segment d'au moins 10 acides aminés adjacents de la SEQ ID n° 2 capable d'être reconnu par des anticorps anti-Anisakis ou
- (ii) L'acide nucléique comprend un fragment de la SEQ ID n° 1 codant pour un segment d'au moins 12 acides aminés adjacents de la SEQ ID n° 2 capable d'être reconnu par des anticorps anti-Anisakis.

3. Un peptide, un polypeptide ou une protéine isolé(e) codé(e) par n'importe laquelle des séquences d'acide nucléique isolées de la revendication 1 et sélectionné(e) dans le groupe suivant :
a. Un peptide, un polypeptide ou une protéine comprenant la SEQ ID n°2.
b. Un fragment de la SEQ ID n° 2 comprenant la SEQ ID n° 16 ou la SEQ ID n°18.

4. Le peptide, le polypeptide ou la protéine isolé(e) est codé(e) par l'acide nucléique de la revendication 2, où
- (i) Le fragment de la SEQ ID n°2 comprend un segment d'au moins 10 acides aminés adjacents capable d'être reconnu par des anticorps anti-Anisakis ou
- (ii) Le fragment de la SEQ ID n°2 comprend un segment d'au moins 12 acides aminés adjacents capable d'être reconnu par des anticorps anti-Anisakis.

5. Le peptide, le polypeptide ou la protéine isolé(e) selon la revendication 3(b) présentant au moins 70 % d'identité avec le fragment de la SEQ ID n° 2 comprenant la SEQ ID n°16 ou la SEQ ID n°18 ; ou selon la revendication 4(ii) présentant au moins 90 % d'identité avec ce fragment.

6. Le peptide, le polypeptide ou la protéine isolé(e) selon la revendication 3(b) présentant au moins 80 % ou au moins 90 % d'identité avec le fragment de la SEQ ID n°2 comprenant la SEQ ID n°16 ou SEQ ID n°18.

7. Un peptide, un polypeptide ou une protéine isolé(e), ou toute séquence d'acide nucléique codant pour la séquence d'acides aminés correspondante, comprenant une séquence d'acides aminés sélectionnée dans le groupe suivant :
a. Une séquence d'acides aminés comprenant la SEQ ID n°7.
b. Une séquence d'acides aminés comprenant la SEQ ID n°8.
c. Une séquence d'acides aminés comprenant la SEQ ID n°9.
d. Une séquence d'acides aminés comprenant la SEQ ID n°10.
e. Une séquence d'acides aminés comprenant la SEQ ID n°11.
f. Une séquence d'acides aminés comprenant la SEQ ID n°12.
g. Une séquence d'acides aminés comprenant la SEQ ID n°13.
h. Une séquence d'acides aminés comprenant la SEQ ID n°14.

8. Un peptide, un polypeptide ou une protéine, ou toute séquence d'acide nucléique codant pour la séquence d'acides aminés correspondante, comprenant au moins deux des séquences d'acides aminés de n'importe laquelle des revendications 3-7.

9. Une séquence d'acide nucléique ou une séquence d'acides aminés isolée selon n'importe laquelle des revendications précédentes, où ladite séquence d'acide nucléique ou d'acides aminés est marquée à l'aide d'une molécule de signalisation.

10. La séquence d'acide nucléique ou la séquence d'acides aminés isolée de selon la revendication 9, où ladite molécule de marquage est sélectionnée dans le groupe suivant :
a. Radioactive
b. Enzymatique
c. Fluorescente
d. Luminescente
e. Chimiluminescente
f. Haptènes
g. Colorée

11. La séquence d'acide nucléique ou la séquence d'acides aminés isolée selon la revendication précédente, où ladite molécule de marquage est sélectionnée dans le groupe suivant :
a. Biotine ou dérivés de la biotine
b. Dérivé du nitrophénol
c. Or colloïdal et
d. Latex

12. Un peptide, un polypeptide ou une protéine isolé(e) selon n'importe laquelle des revendications 3-8, fusionné(e) ou lié(e) chimiquement à un autre peptide ou polypeptide ou à une autre protéine.

13. Un vecteur d'expression ou système d'expression comprenant une séquence nucléotidique selon n'importe laquelle des revendications 1, 2, 5-8.

14. Une cellule hôte procaryote ou eucaryote isolée transformée ou transfectée au moyen d'une séquence d'acide nucléique de n'importe laquelle des revendications 1, 2, 5-11 ou du vecteur ou du système d'expression de la revendication 13.

15. Une méthode de préparation d'un peptide, un polypeptide ou une protéine selon n'importe laquelle des revendications 3-8 ou 12, par l'une des techniques suivantes :
a. Techniques de recombinaison
b. Synthèse chimique
c. Purification substantielle de nématodes de la famille des Anisakidae

16. Une méthode *in vitro* de détection d'anticorps dirigés contre *Anisakis* spp. dans un échantillon biologique qui implique :
a. de placer l'échantillon biologique, de préférence du sérum, en contact avec un peptide, un polypeptide ou une protéine de n'importe laquelle des revendications 3-8 dans des conditions permettant la formation d'un complexe immunologique entre ledit polypeptide et les anticorps de l'échantillon et
b. de détecter ledit complexe immunologique.

17. La méthode de la revendication 16, où ladite méthode est un immunoessai ou un immunoessai enzymatique.

18. Une composition immunologique comprenant un peptide, un polypeptide ou une protéine selon n'importe laquelle des revendications 3-8 ou 12 ou une séquence nucléotidique selon n'importe laquelle des revendications 1, 2, 5-8.

19. Utilisation d'un peptide, un polypeptide ou une protéine selon n'importe laquelle des revendications 3-8 ou 12 dans la fabrication d'un médicament pour le traitement des allergies à *Anisakis* spp.

20. Un kit approprié pour la détection des anticorps *anti-Anisakis* spp. dans un échantillon biologique comprenant un peptide, un polypeptide ou une protéine selon n'importe laquelle des revendications 3-8 ou 12.
